# EUROPEAN PATENT APPLICATION

(11) **EP 4 353 429 A1**
(43) Date of publication of application: **17.04.2024**
(21) Application number: 22819988.1
(22) Date of filing: 11.05.2022
(51) Int. Cl.: B26B 19/14, B26B 19/38, B26B 19/46, B26B 19/48

(54) **SKIN PROCESSING DEVICE**

(30) Priority: 07.06.2021 JP 2021094920
(71) Applicant: Ya-Man Ltd., Tokyo 135-0045 (JP)
(72) Inventor: KIBE Yoichi, Tokyo 135-0045 (JP); MAEDA Kazunori, Tokyo 135-0045 (JP)
(74) Representative: V.O.
(86) International application number: PCT/JP2022/019968
(87) International publication number: WO 2022/259804

(57) **Abstract**

Disclosed is a skin processing device comprising a head part capable of contacting a target region of a user, wherein the head part includes: a motorized cutter unit capable of cutting hair during operation; and an action means for providing, on the basis of electrical power supplied from a power source, a specific action to the target region of the user with which the cutter unit comes in contact. The action means includes, for example, a heating element that produces heat by flowing direct current therethrough and/or a pair of electrodes that generate an electric stimulus.

## Description

### [Technical Field]

The present disclosure relates to a skin treatment device.

### [Background Art]

There has been known a skin treatment device in the form of an electric shaver which is provided with an iontophoresis device in a head part thereof.

### [Prior Art Document]

### [Patent Document]

Patent Document 1: JP 2010-510821A

### [Summary of the Invention]

### [Problems to be Solved by the Invention]

If a skin treatment device such as an electric shaver is capable of applying a specific effect to a target region of a user when its head part comes into contact with the target region, it is possible to expect effects corresponding to the specific effect (for example, an effect of improving hair removal efficiency related to shaving or an effect of appropriately taking care of the skin after shaving).

Accordingly, the present disclosure provides a skin treatment device capable of applying a specific effect to a target region.

### [Means for Solving the Problems]

Provided according to one aspect of the present disclosure is a skin treatment device, including: a head part configured so as to be capable of making contact with a target region of a user. The head part has an electrically powered cutter unit and an effect applicator configured to apply a specific effect to the target region of the user that the head part is in contact with based on electric power supplied from a power source.

### [Effects of the Invention]

According to the present disclosure, a skin treatment device capable of applying a specific effect to a target region can be obtained.

### [Brief Description of the Drawings]

[FIG. 1] FIG. 1 is a front view schematically illustrating an example of an electric shaver according to Embodiment 1.
[FIG. 2] FIG. 2 is a schematic configuration diagram explaining a preferred example of a heater according to Embodiment 1.
[FIG. 2A] FIG. 2A is a schematic diagram illustrating an example of an electrical system of a body part according to Embodiment 1.
[FIG. 3] FIG. 3 is an explanatory diagram of the principle of a warming effect according to Embodiment 1.
[FIG. 4] FIG. 4 is a schematic flowchart illustrating an example of a process performed by a control unit according to Embodiment 1.
[FIG. 5] FIG. 5 is a schematic flowchart illustrating another example of the process performed by the control unit according to Embodiment 1.
[FIG. 6] FIG. 6 is an exploded perspective view of an entire head part according to Embodiment 1.
[FIG. 7] FIG. 7 is an exploded perspective view of a part of the head part according to Embodiment 1.
[FIG. 8] FIG. 8 is a perspective view illustrating a main holder portion alone according to Embodiment 1.
[FIG. 9] FIG. 9 is an explanatory diagram of the warming effect of the electric shaver illustrated in FIGS. 6 to 8 (No. 1).
[FIG. 10] FIG. 10 is an explanatory diagram of the warming effect of the electric shaver illustrated in FIGS. 6 to 8 (No. 2).
[FIG. 11] FIG. 11 is an explanatory diagram of the warming effect of the electric shaver illustrated in FIGS. 6 to 8 (No. 3).
[FIG. 12] FIG. 12 is a plan view schematically illustrating a film heater according to Embodiment 1.
[FIG. 13] FIG. 13 is a diagram illustrating an example of a wiring structure in the head part according to Embodiment 1.
[FIG. 14] FIG. 14 is a diagram illustrating an example of the wiring structure in the head part according to Embodiment 1.
[FIG. 15] FIG. 15 is a perspective view illustrating a held portion of a base portion according to Embodiment 1.
[FIG. 16] FIG. 16 is a perspective view of a head part of an electric shaver according to Embodiment 2.
[FIG. 17] FIG. 17 is another perspective view of the head part of the electric shaver according to Embodiment 2.
[FIG. 18] FIG. 18 is a perspective view of a base portion of the electric shaver according to Embodiment 2.
[FIG. 19] FIG. 19 is an explanatory diagram of a wiring structure according to Embodiment 2.
[FIG. 19A] FIG. 19A is a perspective view from below of a frame according to Embodiment 2.
[FIG. 20] FIG. 20 is a perspective view of an electrical connection structure of the electric shaver according to Embodiment 2.
[FIG. 21] FIG. 21 is an enlarged perspective view of characteristic part of a shaver holder according to Embodiment 2.
[FIG. 22] FIG. 22 is a perspective view illustrating an arrangement of a light irradiation unit, etc. of the electric shaver according to Embodiment 2.
[FIG. 23] FIG. 23 is an explanatory diagram of a method for detaching a cutter unit of the electric shaver according to Embodiment 2.
[FIG. 24] FIG. 24 is a perspective view of the frame of the electric shaver according to Embodiment 2.
[FIG. 25] FIG. 25 is a perspective view from below illustrating a connecting part between the head part and a body part according to Embodiment 2.
[FIG. 26] FIG. 26 is a perspective view of a connection region of the body part according to Embodiment 2.
[FIG. 26A] FIG. 26A is another perspective view of the connection region of the body part according to Embodiment 2.
[FIG. 27] FIG. 27 is a perspective view of a connection region of the head part according to Embodiment 2.
[FIG. 28] FIG. 28 is a perspective view of a second coupling unit according to Embodiment 2.

### [Modes for Carrying Out the Invention]

Exemplary embodiments will now be described in detail with reference to the accompanying drawings.

### [Embodiment 1]

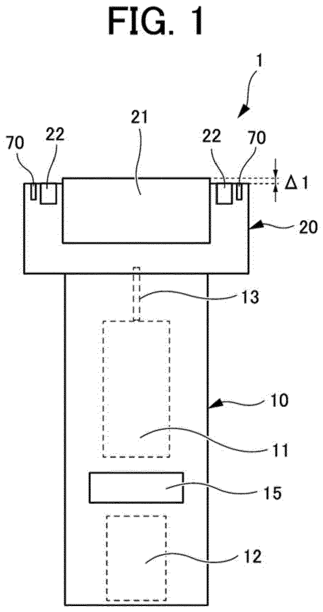

FIG. 1 is a front view schematically illustrating an example of an electric shaver 1 which is one form of a skin treatment device according to Embodiment 1. Hereinafter, a "user" refers to a user of the electric shaver 1, and a "target region" of the user is a region with which the electric shaver 1 can make contact. The target region typically corresponds to a region in which hair to be shaved is present. In addition, the hair removal efficiency means hair removal efficiency related to shaving with the electric shaver 1.

As illustrated in FIG. 1, the electric shaver 1 includes a body part 10 and a head part 20.

The body part 10 accommodates an electric motor 11 and a battery 12. The electric motor 11 is a drive source that drives the electric shaver 1 and operates based on electric power from the battery 12. The electric motor 11 may be a magnetic linear motor or the like. The battery 12 is a battery that can be charged from the outside and functions as an internal power source. In a variant, the electric motor 11 may be operable by an external power source in place of or in addition to the internal battery 12.

Incidentally, the body part 10 may be provided with a user interface 15 that allows various inputs by a user to the electric shaver 1. The user interface 15 may include a mechanical button, a touch switch, or the like. The body part 10 may be provided with a trimmer (not illustrated).

The head part 20 is configured so as to be capable of making contact with a target region of the user. The head part 20 may be detachably attached to the body part 10. When the head part 20 is detachably attachable to the body part 10, cleaning and the like of the head part 20 is facilitated.

The head part 20 may be displaceably supported with respect to the body part 10. When the head part 20 is displaceable with respect to the body part 10, the head part 20 can be easily brought into close contact with the target region of the user, and the hair removal efficiency can be effectively increased. The head part 20 may be displaceable in a variety of degrees of freedom with respect to the body part 10. For example, the head part 20 may be supported with respect to the body part 10 via a shaft member 13, in which case the head part 20 may be tiltable or rotatable within a predetermined angular range about any direction perpendicular to the axial direction of the shaft member 13. Incidentally, a ball joint may be used for a connection structure between the head part 20 and the body part 10.

The head part 20 is provided with a cutter unit 21 and a heater 22.

The cutter unit 21 is provided in the head part 20 in such a manner as to make contact with the target region while the head part 20 is in contact with the target region of the user. That is, the cutter unit 21 forms a region of the head part 20 that makes contact with the target region of the user. In a usage mode for normal shaving, the region of the head part 20 that makes contact with the target region of the user may be only the cutter unit 21.

The cutter unit 21 is of electric type that is driven by the electric motor 11. The cutter unit 21 has a blade that can cut hair during operation (driving). Accordingly, when the cutter unit 21 operates while the head part 20 is in contact with the target region of the user, hair that may be present in the target region is cut by the cutter unit 21 (i.e., shaving is achieved).

Incidentally, the cutter unit 21 may be moved in any manner as long as it is electrically powered. For example, the cutter unit 21 may be of rotary type, oscillation type (linear oscillation), or a combination thereof.

The heater 22 heats the target region of the user that the cutter unit 21 makes contact with. By heating the target region of the user that the cutter unit 21 makes contact with, the heater 22 can help bring the condition of the target region of the user (particularly, the condition of hair) to a condition suitable for shaving. For example, the heater 22 achieves the same effect as warming the target region with a steamed towel. Hereinafter, such an effect of the heater 22 is also referred to as a "warming effect".

The heater 22 operates based on electric power from the battery 12. That is, the power source voltage for the heater 22 is generated based on the battery 12. In a variant, the heater 22 may be operable by an external power source in place of or in addition to the internal battery 12.

The heater 22 may be of any type as long as it is configured to heat the target region. The heater 22 may include, for example, a heating element that generates heat by a DC current flowing therethrough (for example, a heating wire). In this case, the heater 22 may be in the form of a film heater, a nichrome wire heater, or the like.

Alternatively, the heater 22 may be an applicator that applies high frequency to the target region using a pair of electrodes 221 as described below. The heater 22 may be an applicator that irradiates the target region with radio waves such as microwaves (very high frequency radio waves).

The heater 22 is preferably provided over a relatively large area of the head part 20 such that a relatively large area of the target region can be heated while the head part 20 is in contact with the target region of the user. In this case, the heater 22 may be arranged so as to occupy most of the region of the head part 20 that makes contact with the target region of the user excluding the cutter unit 21. For example, the heater 22 may be provided so as to surround the cutter unit 21.

The heater 22 is preferably provided to the head part 20 in a manner that does not inhibit close contact between the cutter unit 21 and the target region. For example, the heater 22 may be configured to be slightly offset with respect to the cutter unit 21 toward the body part 10 side (see Δ1 in FIG. 1). This can reduce the possibility that the adhesion between the cutter unit 21 and the target region decreases due to the heater 22.

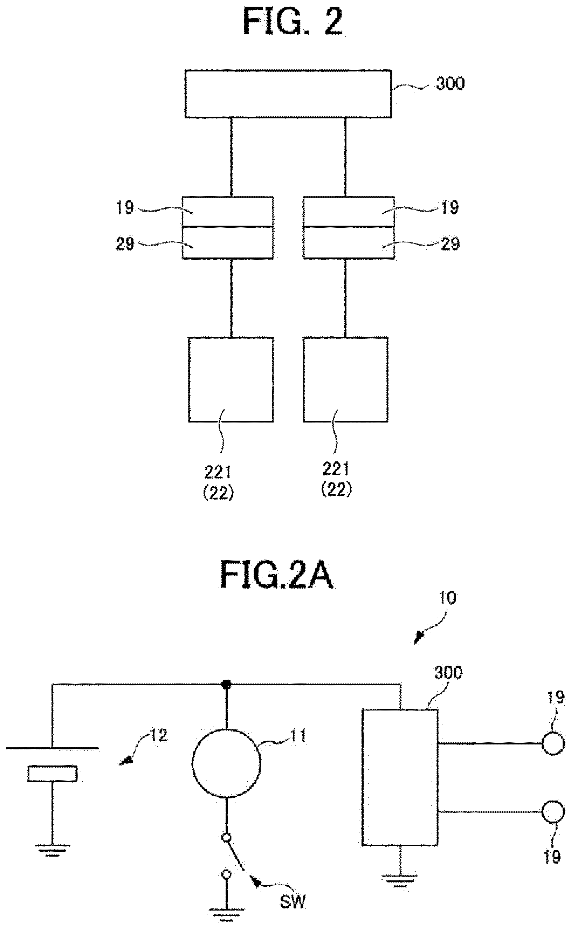

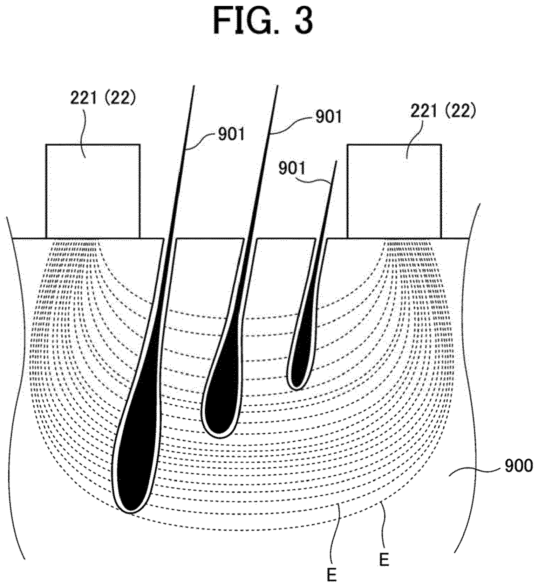

FIG. 2 is a schematic configuration diagram explaining a preferred example of the heater 22. FIG. 2A is a schematic diagram illustrating an example of an electrical system in the body part 10. FIG. 2 also illustrates how the heater 22 and the control unit 300 are electrically connected. The wiring is schematically illustrated by solid lines. FIG. 3 is an explanatory diagram of the principle of the warming effect, and is a cross-sectional view schematically illustrating an electric field (high frequency current) generated in the target region 900 when a high frequency voltage is applied. In FIG. 3, the pair of electrodes 221 as well as three hairs 901 in the target region 900 are schematically illustrated. In addition, FIG. 3 schematically depicts the electric field generated in the target region 900 by application of a high frequency voltage in dotted lines E.

The heater 22 preferably includes the pair of electrodes 221 as illustrated in FIG. 2, and applies a high frequency voltage to the target region 900 of the user via the pair of electrodes 221. In this case, an electric field due to the high frequency voltage is formed between the pair of electrodes 221 in the target region 900, and warming by dielectric warming and/or Joule heat is achieved in the target region 900.

When such a heater 22 is used, the electric field due to a high frequency voltage can apply an effect to relatively deep part of the target region 900 (toward the side close to pores away from the skin surface). Thus, pores can be effectively opened and heat can also be transferred to hair roots to soften the hair 901. Such warming based on the application of a high frequency voltage to the pair of electrodes 221 makes it easier to efficiently bring the condition of the target region 900 of the user (in particular, the condition of hair) to a condition suitable for shaving as compared with the case where a heating element that generates heat by DC current supplied is used. That is, the warming effect can be efficiently increased.

When the heater 22 includes the pair of electrodes 221, one of the pair of electrodes 221 may be formed by a component of the cutter unit 21 (an outer blade portion 2131 as described later).

In the example illustrated in FIG. 2, the pair of electrodes 221 on the head part 20 side is electrically connected to the control unit 300 on the body part 10 side (and to the battery 12 not illustrated in FIG. 2) via electrical connection between electrical contacts 19 on the body part 10 side and electrical contacts 29 on the head part 20 side. This arrangement eliminates the necessity of providing the control unit 300 and a battery (for example, a battery other than the battery 12) on the head part 20 side, enabling the cleaning of the head part 20 and the like. The electrical contacts 19 and the electrical contacts 29 may be provided in pairs corresponding to the pair of electrodes 221.

The electrical contacts 19 and the electrical contacts 29 may be disposed, for example, at a connection portion between the head part 20 and the body part 10. For example, the electrical contacts 19 on the body part 10 side may be provided at or near a holding portion of the body part 10 that detachably holds the head part 20, and the electrical contacts 29 on the head part 20 side may be provided at or near a held portion of the head part 20. In this case, the electrical contacts 19 and the electrical contacts 29 are electrically connected when the head part 20 is connected (attached) to the body part 10, and are electrically disconnected when the head part 20 is separated from the body part 10.

Alternatively, the electrical contacts 19 and the electrical contacts 29 may be disposed at positions other than the connection portion between the head part 20 and the body part 10 (positions apart from the holding portion described above). For example, the electrical contacts 19 and the electrical contacts 29 may be magnet contacts that are brought into contact when the head part 20 is connected (attached) to the body part 10. Alternatively, energization between the electrical contacts 19 and the electrical contacts 29 may be contactless energization (non-contact type energization). Alternatively, the electrical contacts 19 may be omitted and the electrical contacts 29 may be connectable to an external power source. In this case, the electrical connection between the electrical contacts 29 and the external power source may be a USB (Universal Serial Bus) connection or the like.

In the present embodiment, the control unit 300 may be electrically connected to the battery 12 in parallel with the electric motor 11 as illustrated in FIG. 2A. In this way, the heater 22 can be operated utilizing electric power of the battery 12, which is a power source for the electric motor 11. Incidentally, in FIG. 2A, a switch SW is a shaving switch for turning on the electric motor 11 (an element of the user interface 15).

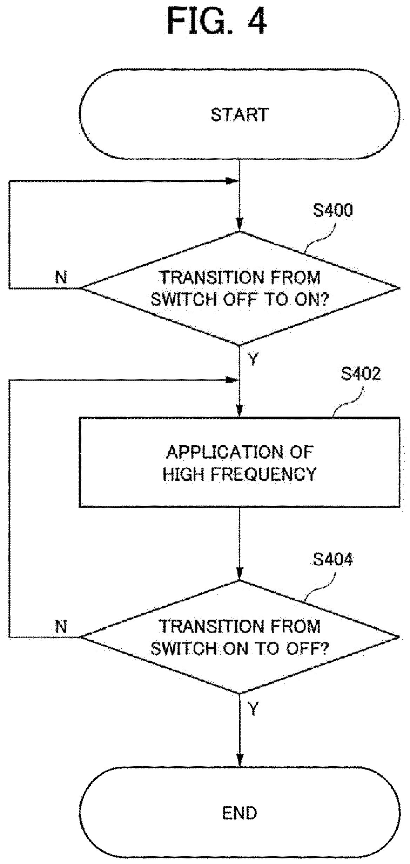

FIG. 4 is a schematic flowchart illustrating an example of a process performed by the control unit 300.

In the example illustrated in FIG. 4, the heater 22 operates in conjunction with the operation of the cutter unit 21 at all times. Specifically, when the shaving switch (not illustrated) is turned on (YES in step S400), electric power supply to the electric motor 11 and the control unit 300 is started. In this case, when the cutter unit 21 is rotated by the actuation of the electric motor 11, the control unit 300 starts applying a high frequency voltage to the pair of electrodes 221 related to the heater 22 (step S402). In this case, the user can operate the heater 22 without a particular operation to the heater 22 when shaving with the cutter unit 21, thereby improving the operability. When the shaving switch (not illustrated) is turned off (YES in step S404), the electric power supply from the battery 12 is shut off to stop the electric motor 11 and the heater 22. That is, when the electric motor 11 is stopped, the cutter unit 21 and the application of a high frequency voltage to the pair of electrodes 221 related to the heater 22 are both stopped.

In the example illustrated in FIG. 4, the operation of the heater 22 starts at substantially the same timing as the start of the operation of the electric motor 11, but a significant shift may be set between the start timing of the operation of the electric motor 11 and the start timing of the operation of the heater 22. For example, the start timing of the operation of the heater 22 may be significantly earlier than the start timing of the operation of the electric motor 11 such that shaving is performed in a warmed target region.

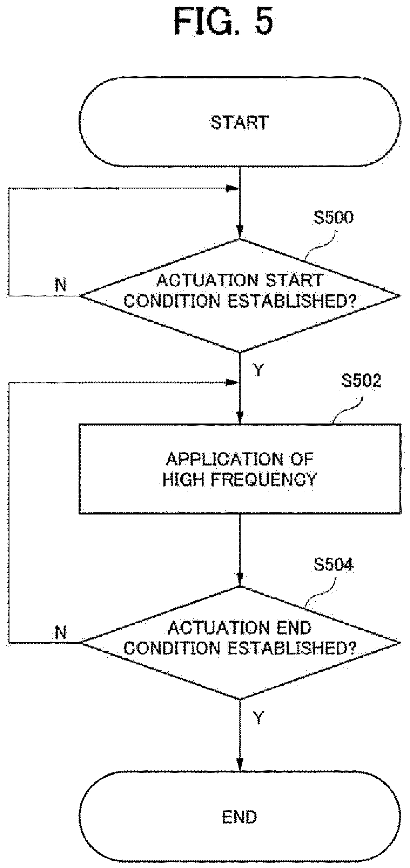

FIG. 5 is a schematic flowchart illustrating another example of the process performed by the control unit 300.

In the example illustrated in FIG. 5, the heater 22 is operable independently of the operation of the cutter unit 21. Specifically, in step S500, the control unit 300 determines whether or not an actuation start condition of the heater 22 has been established. The actuation start condition of the heater 22 may be any condition and may vary. For example, the actuation start condition of the heater 22 may be satisfied by turning on a heating switch (not illustrated). In such a configuration where the heating switch (not illustrated) is provided separately from the shaving switch (see the switch SW in FIG. 2A), the heater 22 can operate independently of the electric motor 11, which is actuated by turning on the shaving switch.

The control unit 300 starts operating the heater 22 by the establishment of the actuation start condition of the heater 22 (YES in step S500), and subsequently determines whether or not an actuation end condition of the heater 22 has been established (step S502). The actuation end condition of the heater 22 may be any condition and may vary. For example, the actuation end condition of the heater 22 may be satisfied by turning off the heating switch (not illustrated). When the actuation end condition of the heater 22 is satisfied (YES in step S504), the control unit 300 stops applying a high frequency voltage to the pair of electrodes 221 related to the heater 22 (step S506).

In an alternative embodiment, a variety of operation modes may be provided. For example, the plurality of operation modes may include a shaving-only mode in which only the electric motor 11 is actuated, a shaving and heating mode in which both the electric motor 11 and the heater 22 are actuated, and a heating-only mode in which only the heater 22 is actuated.

In an alternative embodiment, the head part 20 may be provided with a temperature sensor that detects the temperature of the heater 22 or the target region, in which case the control unit 300 may control the heater 22 based on temperature information from the temperature sensor. For example, the control unit 300 may control the heater 22 such that the temperature indicated by the temperature information from the temperature sensor matches a predetermined target value. In this case, the predetermined target value may be a fixed value, or the user may be able to select one from a plurality of stages or adjust (customize) it.

In an alternative embodiment, the head part 20 or the body part 10 may be provided with a current sensor. In this case, when the temperature or the current exceeds a predetermined threshold value, the degree of heating by the heater 22 may be decreased or the heating by the heater 22 may be stopped. On the other hand, when the temperature or the current is below a threshold value, the degree of heating by the heater 22 may be increased. The temperature sensor or the current sensor may be capable of detecting that a foreign object has adhered to the head part 20. If desired improvement in skin quality cannot be obtained, the degree of operation of the electric shaver 1 may be reduced or the electric shaver 1 may be stopped.

In an alternative embodiment, the head part 20 may be provided with a contact or proximity sensor that detects a contact or the proximity of the head part 20 with respect to the target region, in which case the control unit 300 may control the heater 22 based on sensor information from the contact or proximity sensor. For example, the control unit 300 may actuate the heater 22 only if the sensor information from the contact or proximity sensor indicates a contact or the proximity between the head part 20 and the target region.

Since the heater 22 is provided to the head part 20 in the present embodiment, a configuration capable of informing the user of an operation status of the heater 22 and the like can be useful. Particularly in a configuration where the heater 22 and the electric motor 11 do not operate in conjunction with each other and can operate independently as illustrated in FIG. 5, informing the user of the operation status of the heater 22 increases the convenience.

Accordingly, in the present embodiment, the head part 20 may be provided with a light irradiation unit 70 (see FIG. 1). The light irradiation unit 70 may be configured to irradiate light for transfer of information. For example, in the case where a plurality of operation modes is provided as described above in reference to FIG. 5, the light irradiation unit 70 may irradiate light in different colors for each operation mode. The light irradiation unit 70 may be formed by a light-emitting diode or the like. The light irradiation unit 70 may be configured to provide light stimulation to the skin in accordance with the frequency of the light it irradiates, or may be disposed such that the user can see the state of the skin subject to treatment in visible light it irradiates.

Alternatively, the light irradiation unit 70 may function as a heater different from the heater 22. Alternatively, the light irradiation unit 70 may irradiate light with a frequency that acts to damage hair roots. In this case, turning on and off of the light irradiation unit 70 as well as the output of the light irradiation unit 70 while the light irradiation unit 70 is on may be adjusted (customized) by the user.

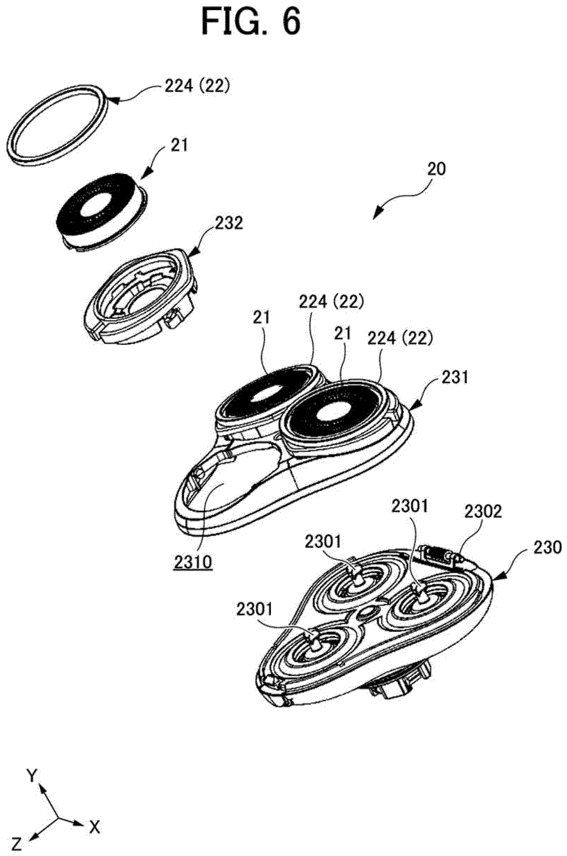

Next, an example of the head part 20 will be described in detail with reference to FIG. 6 and subsequent figures.

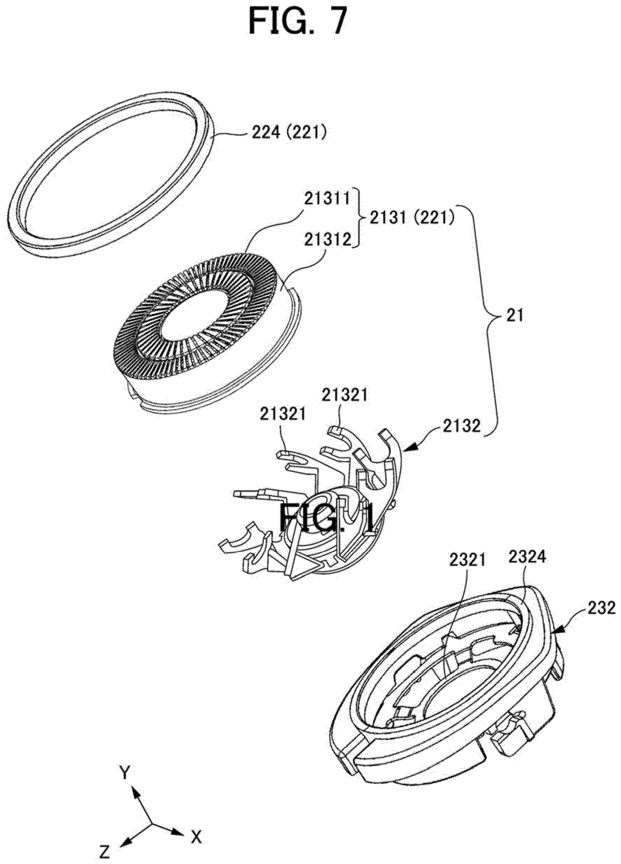

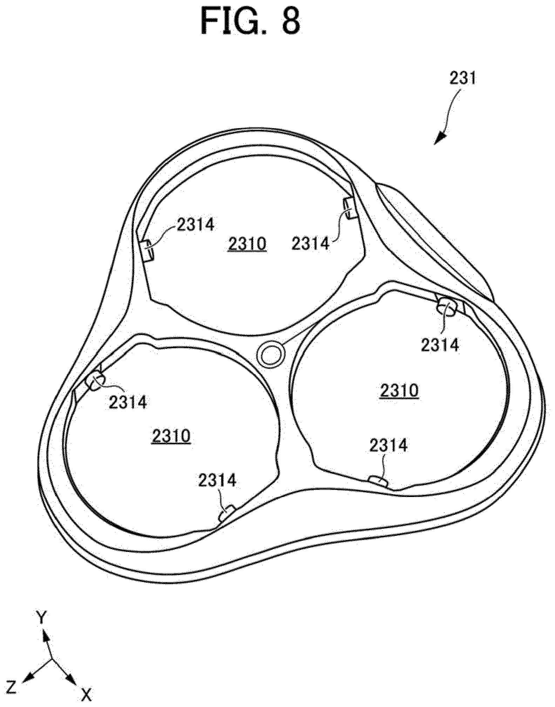

FIG. 6 is an exploded perspective view of the entire head part 20, and FIG. 7 is an exploded perspective view of a part of the head part 20. FIG. 8 is a perspective view illustrating a main holder portion 231 alone. FIGS. 6 to 8 each include a coordinate system on the left-hand side, in which three orthogonal axes (X-axis, Y-axis, and Z-axis) are indicated.

In the example illustrated in FIGS. 6 to 8, the head part 20 includes the cutter unit 21 and the heater 22, and the cutter unit 21 and the heater 22 are each provided in three sets. In a variant, the number of sets may be a number other than three (for example, one set, two sets, or four or more sets). In the example illustrated in FIGS. 6 to 8, the pair of electrodes 221 of the heater 22 is formed by an outer blade portion 2131 and a frame 224, as described later. The electrical connection between the three sets of heaters 22 and the control unit 300 (and the battery 12) may be implemented by the electrical contacts 19, 29 as illustrated in FIG. 2. In this case, although six sets of the electrical contact 19 and the electrical contact 29 may be provided for a total of six electrodes 221 related to the three sets of heaters 22, it is preferred that two sets of electrical contacts 19, 29 are provided. In the case of the two sets of electrical contacts 19, 29, the six electrodes 221 related to the three sets of heaters 22 operate in common. This arrangement can simplify the electrical contact structure between the body part 10 and the head part 20.

In the example illustrated in FIGS. 6 to 8, the head part 20 includes a base portion 230, the main holder portion 231 (an example of a holder portion), and a shaver holder 232. In the example illustrated in FIGS. 6 to 8, the shaver holder 232 is provided in three sets corresponding to the three sets of cutter units 21, together with the frames 224 described later. In FIG. 6, only one set of the three sets is disassembled from the main holder portion 231. The three sets may have the same configuration, and any one set will be described below unless otherwise noted.

The base portion 230 can be removably connected to the body part 10 (see FIG. 1). The base portion 230 may be supported with respect to the body part 10 in such a manner that the head part 20 is displaceable with respect to the body part 10 as described above.

The base portion 230 has a rotation shaft portion 2301 that rotates in accordance with the rotation from the electric motor 11. The rotation shaft portion 2301 may be provided for each cutter unit 21. Consequently, in the example illustrated in FIGS. 6 to 8, three rotation shaft portions 2301 are provided. Each of the rotation shaft portions 2301 may be coupled to the shaft member 13 (see FIG. 1) that rotates integrally with the rotation shaft of the electric motor 11 via a power transmission mechanism (not illustrated) such as a gear in such a manner that the rotation shaft portions 2301 can transmit power.

The main holder portion 231 holds the cutter unit 21 and the frame 224 via the shaver holder 232. The main holder portion 231 has an opening 2310 in which the shaver holder 232 is disposed.

The main holder portion 231 is attached to the base portion 230. The main holder portion 231 may be fixed with respect to the portion 230, but it is preferred that the main holder portion 231 is removably or openably and closably supported with respect to the base portion 230. In the example illustrated in FIGS. 6 to 8, the main holder portion 231 is supported rotatably (openably and closably) about an opening and closing shaft 2302 of the base portion 230. In this case, the main holder portion 231 can be opened and closed with respect to the base portion 230 by its rotation about the opening and closing shaft 2302. The main holder portion 231 is closed in normal use and may be opened to remove hair (hair that has been cut off) that may accumulate between the main holder portion 231 and the base portion 230.

The shaver holder 232 is formed of, for example, a resin or the like. The shaver holder 232 holds the cutter unit 21 and the frame 224. The shaver holder 232 may have a stopper 2321 that restricts movement of the cutter unit 21 toward the negative side in the Y direction.

The shaver holder 232 is attached to the main holder portion 231 as can be seen in FIG. 6. The shaver holder 232 may be integrally secured to the main holder portion 231, but is preferably displaceably supported with respect to the main holder portion 231. This makes it easy to bring the cutter unit 21 into close contact with the target region of the user, and the hair removal efficiency can be effectively increased. In the example illustrated in FIGS. 6 to 8, as illustrated in FIG. 8, the main holder portion 231 has rotation shaft support portions 2314 extending in a direction perpendicular to the corresponding rotation shaft portion 2301, and the main holder portion 231 supports the shaver holders 232 rotatably about the rotation shaft support portions 2314.

In the present embodiment, the cutter unit 21 is supported by the main holder portion 231 via the shaver holder 232 as described above. The cutter unit 21 includes the outer blade portion 2131 and an inner blade portion 2132 that is of rotary type as illustrated in FIG. 7.

The outer blade portion 2131 has, on the positive side in the Y direction, a contact surface portion 21311 that can make contact with the target region. A plurality of fine holes, into which hair can enter, are formed in the contact surface portion 21311, and rims of the holes form an outer blade. The contact surface portion 21311 has a function to stand hair up by receiving the hair in the fine holes. When hair is made stand up, the hair can be easily cut by a combination of the outer blade portion 2131 and the inner blade portion 2132. In the example illustrated in FIGS. 6 to 8, the outer blade portion 2131 has a peripheral wall portion 21312, which is cylindrical, and the positive side of the peripheral wall portion 21312 in the Y direction is covered by the contact surface portion 21311. The inner blade portion 2132 is accommodated on the inner diameter side of the cylindrical peripheral wall portion 21312.

In the present embodiment, the outer blade portion 2131 is a conductor and is formed of a conductive material. The outer blade portion 2131 forms one of the pair of electrodes 221 of the heater 22 described above with reference to FIGS. 2 and 3. Specifically, the contact surface portion 21311 that makes contact with the target region 900 of the outer blade portion 2131 forms one of the pair of electrodes 221 of the heater 22 described above with reference to FIGS. 2 and 3.

The outer blade portion 2131 may be formed of one or more materials, or may be formed of two or more materials. For example, the contact surface portion 21311 may be formed of a relatively rigid conductive material such as a metal, and the peripheral wall portion 21312 may be formed of an insulating material such as a resin.

The inner blade portion 2132 is non-rotatably connected to the rotation shaft portion 2301 and integrally rotates with the rotation shaft portion 2301. The inner blade portion 2132 is driven by the electric motor 11 so as to rotate about the rotation shaft of the rotation shaft portion 2301. The inner blade portion 2132 has a plurality of cutter blades 21321 with cutting edges. The plurality of cutter blades 21321 is disposed adjacent in the Y direction to the contact surface portion 21311 of the outer blade portion 2131. In this case, when the inner blade portion 2132 rotates, the hair 901 passing through the holes of the contact surface portion 21311 is cut by the outer blade and the cutter blades 21321 of the inner blade portion 2132.

The frame 224 forms an electrode ring which is provided around the cutter unit 21. That is, the frame 224 is disposed on the outer diameter side of the cylindrical peripheral wall portion 21312 of the outer blade portion 2131. The frame 224 is supported by the main holder portion 231 via the shaver holder 232 as described above.

In the example illustrated in FIGS. 6 to 8, the frame 224 is a conductor and is formed of a conductive material. Specifically, the frame 224 forms the other of the pair of electrodes 221 of the heater 22 described above with reference to FIGS. 2 and 3. That is, the frame 224 forms the other of the pair of electrodes 221 of the heater 22 described above with reference to FIGS. 2 and 3 in cooperation with the contact surface portion 21311 of the outer blade portion 2131.

By implementing the pair of electrodes 221 of the heater 22 by the frame 224 and the contact surface portion 21311 of the outer blade portion 2131, the warming effect can be applied over the entire area in contact with the contact surface portion 21311 out of the target region.

The frame 224 is slightly spaced with respect to the outer blade portion 2131 such that no electrical short circuit occurs between the frame 224 and the outer blade portion 2131. In the example illustrated in FIGS. 6 to 8, the frame 224 has an inner diameter larger than an outer diameter of the cylindrical peripheral wall portion 21312, and the frame 224 is radially spaced with respect to the peripheral wall portion 21312. The shaver holder 232 has a ring-shaped peripheral wall 2324 protruding in the Y direction, and the peripheral wall 2324 is disposed between the frame 224 and the outer blade portion 2131 in the radial direction. This reliably prevents an electrical short circuit between the frame 224 and the outer blade portion 2131.

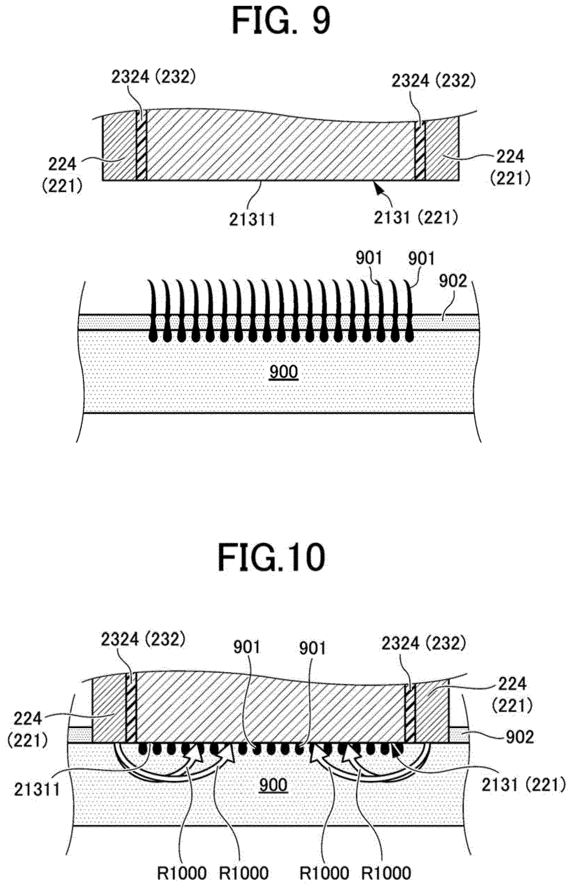

FIGS. 9 to FIG. 11 are explanatory diagrams of the warming effect of the electric shaver 1 according to the example illustrated in FIGS. 6 to 8, and FIGS. 9 to FIG. 11 are cross-sectional views schematically illustrating only an edge part (an edge on the positive side in the Y direction) of the electric shaver 1 in relation to the target region.

FIG. 9 illustrates a state in which the head part 20 of the electric shaver 1 is right before coming into contact with the target region 900. In the example illustrated in FIG. 9, the target region 900 has a large number of hairs 901 to be shaved, and a shaving gel 902 is applied on the target region 900. In place of the shaving gel 902, a shaving foam, a lotion, or the like may be used. The use of the shaving gel 902 or the like can efficiently increase the warming effect of the application of a high frequency voltage. In addition, since the pores open up and moisture penetrates into and around the pores, the hairs 901 become softer, which facilitates shaving of the hairs 901 (i.e., reduces hairs left unshaved). Further, the moisture penetration into the target region 900 can reduce shaving irritation.

FIG. 10 illustrates a state in which the head part 20 of the electric shaver 1 has just come into contact with the target region 900. In the example illustrated in FIGS. 6 to 8, a high frequency voltage is applied to the target region 900 with the frame 224 as an outer electrode 221 and the contact surface portion 21311 as an inner electrode 221. In FIG. 10, paths of high frequency current are schematically represented by arrows R1000.

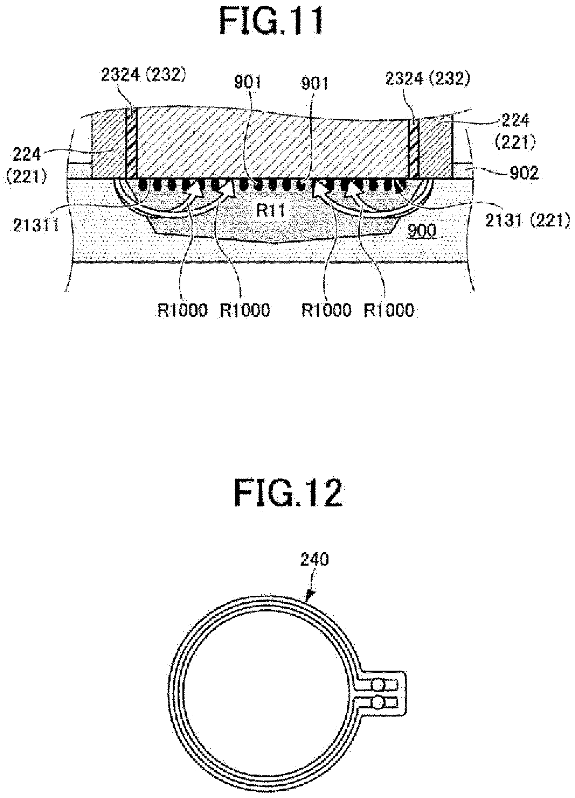

FIG. 11 illustrates a state in which the head part 20 of the electric shaver 1 is in contact with the target region 900 and the heater 22 has started operating. In FIG. 11, an area being warmed is schematically represented by a hatched region R11. In this way, according to the example illustrated in FIGS. 6 to 8, the high frequency current can be applied over the entire area in contact with the contact surface portion 21311 out of the target region. As a consequence, the warming effect by the heater 22 can be efficiently applied over a relatively wide area of the target region. In addition, the electric warming effect based on the high frequency current by the heater 22 is less likely to cause drying of the skin as compared with warming from the outside such as a heater or light irradiation, so that an effect to keep the skin hydrated can be expected.

Thus, according to the example illustrated in FIGS. 6 to 8, the electric shaver 1 having the configuration for warming the target region 900 can efficiently provide the warming effect over a relatively large area of the target region 900. As a result, it is possible to effectively increase the hair removal efficiency.

Further, according to the example illustrated in FIGS. 6 to 8, since one of the pair of electrodes 221 of the heater 22 is implemented by the outer blade portion 2131 (the contact surface portion 21311) while the other of the pair of electrodes 221 is implemented by the frame 224, the heater 22 can be composed of a relatively small number of components. That is, by utilizing the outer blade portion 2131, which is an existing component, the number of components related to components that are required for providing the heater 22 can be reduced.

In the example illustrated in FIGS. 6 to 8, one of the pair of electrodes 221 of the heater 22 is implemented by the entire contact surface portion 21311, but may be implemented by only part of the contact surface portion 21311.

Similarly, the other of the pair of electrodes 221 of the heater 22 is implemented by the entire frame 224, but may be implemented by only part of the frame 224. For example, the frame 224 may be formed by insert molding using a resin and a metal material. In this case, the region of the metal material forms the electrode 221.

In the example illustrated in FIGS. 6 to 8, the light irradiation unit 70 described above with reference to FIG. 1 may be provided around the cutter unit 21, that is, to the frame 224. Alternatively, the light irradiation unit 70 may be provided on the outer side of the frame 224. Alternatively, the light irradiation unit 70 may be provided at the end of the peripheral wall 2324 on the positive side of the Y direction.

In the example illustrated in FIGS. 6 to 8, the frame 224 may be provided with, as an alternative heater 22, a film heater 240 as schematically illustrated in a plan view in FIG. 12. In this case, the film heater 240 may be disposed to the frame 224 in such a manner as not to inhibit the function of the frame 224 as the electrode 221. Alternatively, in this case, the pair of electrodes 221 may be omitted and the film heater 240 may function as the only heater 22. Also in the case that the film heater 240 is used, the wiring from the battery 12 or the control unit 300 may be as described above with reference to FIG. 2.

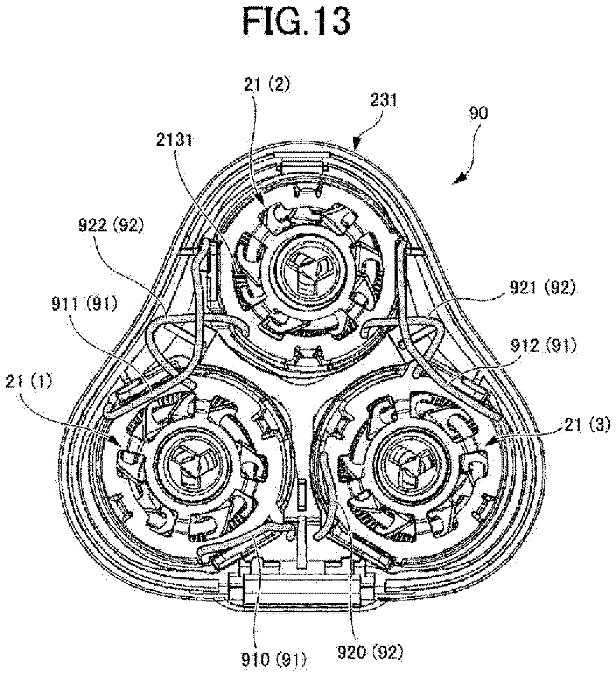

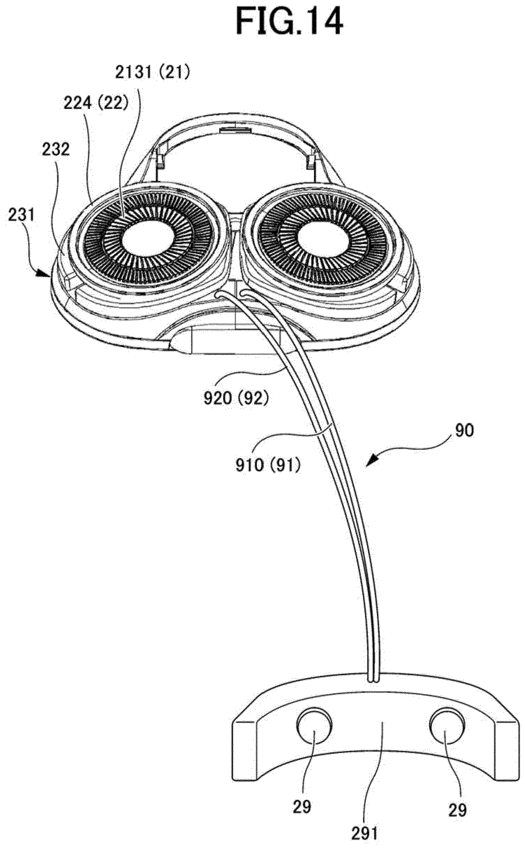

Next, an exemplary wiring structure 90 of the head part 20 that is applicable to the example illustrated in FIGS. 6 to 8 will be described with reference to FIGS. 13 and 14. In the following description, the three cutter units 21 are identified by (1), (2) and (3) which are added at the end of the reference sign when the three cutter units 21 need to be distinguished. In FIG. 14, one of the cutter units 21 is removed and not illustrated.

FIG. 13 and FIG. 14 are diagrams illustrating an example of the wiring structure 90 of the head part 20.

The wiring structure 90 is an electrical connection structure that establishes an electrical connection between the pair of electrical contacts 29 and the pair of electrodes 221 illustrated in FIG. 2. The wiring structure 90 includes a contact retaining portion 290 that retains the pair of electrical contacts 29 as illustrated in FIG. 14. The contact retaining portion 290 is removably attachable to a contact retaining portion on the body part 10 side (not illustrated). In this case, a magnet contact using magnets may be employed. When the contact retaining portion 290 is attached to the contact retaining portion on the body part 10 side, the pair of electrical contacts 29 are electrically connected to the pair of electrical contacts 19 (see FIG. 2).

The wiring structure 90 includes a first wiring 91 (an exemplary wiring) having one end joined to one of the pair of electrical contacts 29 and a second wiring 92 having one end joined to the other of the pair of electrical contacts 29. The other end of the first wiring 91 is joined to one of the pair of electrodes 221, and the other end of the second wiring 92 is joined to the other of the pair of electrodes 221.

As illustrated in FIG. 13, the first wiring 91 includes a first lead-out portion 910 and two first bridge portions 911, 912 (an exemplary wiring portion).

The first lead-out portion 910 is passed from the outside to the inside of the main holder portion 231. The first lead-out portion 910 has one end that is outside the main holder portion 231 and joined to one of the pair of electrical contacts 29 as illustrated in FIG. 14; and the other end that is inside the main holder portion 231 and joined to the outer blade portion 2131 of one cutter unit 21(1). The first lead-out portion 910 may be joined to the lower side (the negative side in the Y direction) of the outer blade portion 2131.

As illustrated in FIG. 13, the first bridge portion 911 has one end joined to the outer blade portion 2131 of the cutter unit 21(1); and the other end joined to the outer blade portion 2131 of another cutter unit 21(2). In this way, the outer blade portion 2131 of the cutter unit 21(1) and the outer blade portion 2131 of the cutter unit 21(2) are electrically connected via the first bridge portion 911.

As illustrated in FIG. 13, the first bridge portion 912 has one end joined to the outer blade portion 2131 of the cutter unit 21(2); and the other end joined to the outer blade portion 2131 of further another cutter unit 21(3). In this way, the outer blade portion 2131 of the cutter unit 21(2) and the outer blade portion 2131 of the cutter unit 21(3) are electrically connected via the first bridge portion 912.

Thus, the first wiring 91 electrically connects one of the pair of electrical contacts 29 to the respective outer blade portions 2131 of the three cutter units 21 that form one of the pair of electrodes 221 via the first lead-out portion 910 and the two first bridge portions 911, 912. This allows the three cutter units 21 forming one of the pair of electrodes 221 to be energized all at once via one of the pair of electrical contacts 29.

Similarly, the second wiring 92 includes a second lead-out portion 920 and two second bridge portions 921, 922.

The second lead-out portion 920 is passed from the outside to the inside of the main holder portion 231. The second lead-out portion 920 has one end that is outside the main holder portion 231 and joined to the other of the pair of electrical contacts 29 as illustrated in FIG. 14; and the other end that is inside the main holder portion 231 and joined to the frame 224 around the cutter unit 21(3). The second lead-out portion 920 may be joined to the lower side (the negative side in the Y direction) of the frame 224.

As illustrated in FIG. 13, the second bridge portion 921 has one end joined to the frame 224 around the cutter unit 21(3); and the other end joined to the frame 224 around the cutter unit 21(2). In this way, the frame 224 around the cutter unit 21(3) and the frame 224 around the cutter unit 21(2) are electrically connected via the second bridge portion 921.

As illustrated in FIG. 13, the second bridge portion 922 has one end joined to the frame 224 around the cutter unit 21(2); and the other end joined to the frame 224 around the cutter unit 21(1). In this way, the frame 224 around the cutter unit 21(2) and the frame 224 around the cutter unit 21(1) are electrically connected via the second bridge portion 922.

Thus, the second wiring 92 electrically connects the other of the pair of electrical contacts 29 to the respective three frames 224 each forming the other of the pair of electrodes 221 via the second lead-out portion 920 and the two second bridge portions 921, 922. This allows the three frames 224 each forming the other of the pair of electrodes 221 to be energized all at once via the other of the pair of electrical contacts 29.

Note that, in the example illustrated in FIGS. 6 to 8, the cutter units 21 are supported so as to be displaceable with respect to the main holder portion 231 as described above. Hence, the cutter units 21 each can be displaced with respect to the main holder portion 231 in accordance with the shape of the target region 900 and the like during the use of the electric shaver 1.

Accordingly, in the wiring structure 90 illustrated in FIGS. 13 and 14, the first lead-out portion 910 of the first wiring 91 is preferably configured so as to maintain a state in which the first lead-out portion 910 is joined with one of the pair of electrodes 221 even when the cutter unit 21 is displaced with respect to the main holder portion 231. Specifically, it is preferred that the first lead-out portion 910 of the first wiring 91 is moderately slack or has flexibility in order to absorb the displacement of the cutter unit 21(1) with respect to the main holder portion 231. For example, the first lead-out portion 910 may be composed of an electric wire including a single wire or a strand wire, a flat cable, a conductive spring, or the like. This enables stable heating of the skin of the target portion 900 even when the cutter unit 21 (1) is displaced in accordance with the unevenness of the target portion 900 during the use of the electric shaver 1.

Similarly, in the wiring structure 90 illustrated in FIGS. 13 and 14, the first bridge portion 911 of the first wiring 91 is preferably configured so as to maintain a state in which the first bridge portion 911 is joined with one of the pair of electrodes 221 at both ends even when the two cutter units 21 are displaced independently of each other with respect to the main holder portion 231. Specifically, it is preferred that the first bridge portion 911 of the first wiring 91 is moderately slack or has flexibility in order to absorb the independent displacement of each of the two cutter units 21 with respect to the main holder portion 231. For example, the first bridge portion 911 may be composed of an electric wire including a single wire or a strand wire, a flat cable, a conductive spring, or the like. This enables stable heating of the skin of the target portion 900 even when the two cutter units 21(1)(2) are displaced independently of each other in accordance with the unevenness of the target portion 900 during the use of the electric shaver 1. This also applies to the first bridge portion 912.

Since the frames 224 are not displaceable with respect to the main holder portion 231 unlike the cutter units 21, the second wiring 92 may be configured so as to be relatively a little slack or have low flexibility.

According to the example illustrated in FIGS. 13 and 14, even in the case of the head part 20 having the three cutter units 21, providing the first bridge portions 911, 912 and the second bridge portions 921, 922 makes it possible to achieve the wiring structure 90 that is efficient with regard to the pairs of electrodes 221, each pair of which is provided in correspondence with each of the three cutter unit 21.

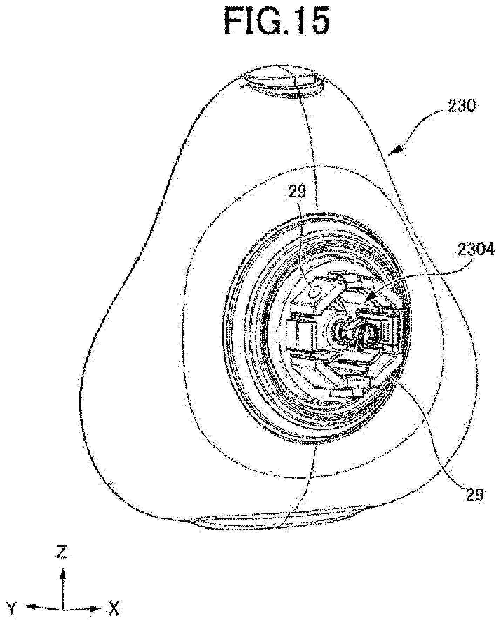

In the example illustrated in FIGS. 13 and 14, the pair of electrical contacts 29 on the head part 20 side is retained in the contact retaining portion 290 which is different from the connection portion between the head part 20 and the body part 10. However, the arrangement is not limited thereto. For example, the pair of electrical contacts 29 on the head part 20 side may be provided at the connection portion between the head part 20 and the body part 10, as described above. For example, the pair of electrical contacts 29 on the head part 20 side may be provided to the held portion 2304 of the base portion 230 as illustrated in FIG. 15. In this case, the first lead-out portion 910 of the first wiring 91 and the second lead-out portion 920 of the second wiring 92 may be drawn to the base portion 230 using the rotation shaft portion 2301 or the like. In this instance, a part of the first lead-out portion 910 of the first wiring 91 and a part of the second lead-out portion 920 of the second wiring 92 may each be a conductor portion that may be embedded in the base portion 230 by insert molding or the like.

In addition, in the example illustrated in FIGS. 13 and 14, a part of the first wiring 91 and a part of the second wiring 92 may each be formed by a conductive portion that may be embedded in the main holder portion 231 by insert molding or the like.

In the present embodiment, the first wiring 91 and/or the second wiring 92 may be composed of a wire made of stainless steel. In addition, the first wiring 91 and/or the second wiring 92 may be plated with nickel or zinc. By plating the surface of stainless steel that has surface corrosion resistance and high electrical resistance due to a passive film with nickel having low electrical resistance, the first wiring 91 and/or the second wiring 92 become rust resistant and capable of easily passing electricity therethrough. The ability to pass electricity on the premise of water washing facilitates the designing. The electrical contacts 19 and/or the electrical contacts 29 may also be composed of plated stainless steel as well. The surfaces of the first wiring 91 and/or the second wiring 92 as well as the electrical contacts 19 and/or the electrical contacts 29 may be made of the same material such as nickel. Degradation due to dissimilar metal contact corrosion can be prevented.

In the present embodiment, the first wiring 91 and the second wiring 92 may be provided with an insulation film on the surfaces thereof or may have a wide separation distance therebetween to prevent a contact and hence an electrical short circuit between the first wiring 91 and second wiring 92. The entire head part 20 may be downsized while a wall is provided between the wirings to increase the creepage distance.

Further, in the present embodiment, it may be arranged in such a way that the insulation resistance of the first wiring 91 and/or the second wiring 92 is larger than the insulation resistances between the frames 224 and the respective outer blade portions 2131. For example, the distance may be large. This allows more current to flow toward the frames 224 and the outer blade portions 2131 even when an area between the first wiring 91 and the second wiring 92 and areas between the frames 224 and the respective outer blade portions 2131 are filled with a conductive material such as a liquid including water or a gel. For example, the battery 12 can be downsized while ensuring skin heating with high frequency by arranging such that the insulating resistances of the first wiring 91 and the second wiring 92 are 1 megohm or more when they are dry and 100 ohm or more when they are filled with a gel.

### [Embodiment 2]

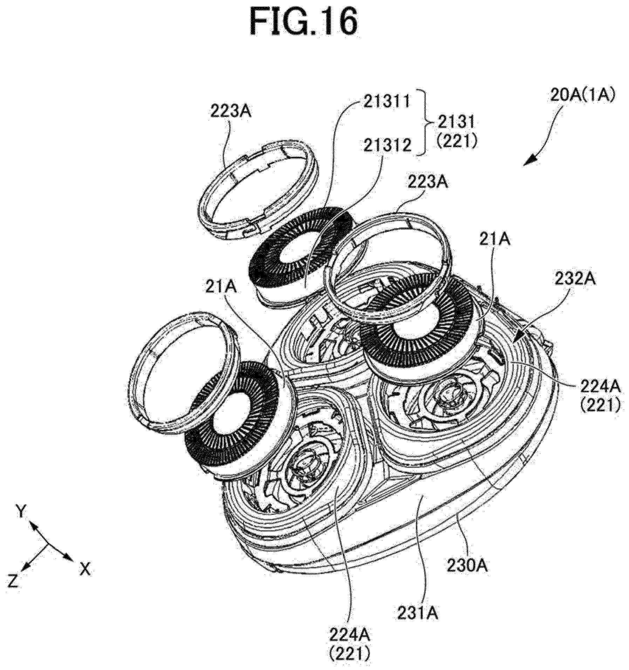

Next, Embodiment 2, which can be suitably implemented in place of Embodiment 1, will be described with reference to FIG. 16 and subsequent figures. In the following description and the figures of Embodiment 2, components that may be similar to those in Embodiment 1 described above may be denoted by similar reference signs to omit the description thereof. In addition, in the following description, a side close to the skin of a user of a head part 20A when an electric shaver 1A is in use (i.e., the positive side in the Y direction) may be referred to as "the upper side", and the opposite side thereof, that is, a side far from the skin (i.e., the negative side in the Y direction) may be referred to as "the lower side".

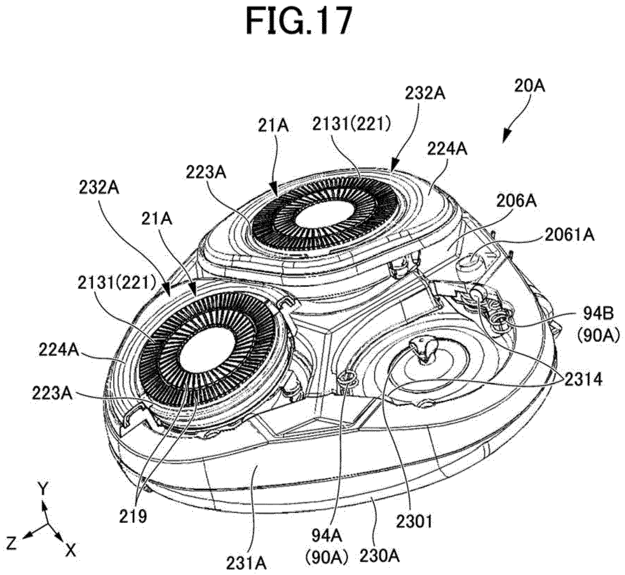

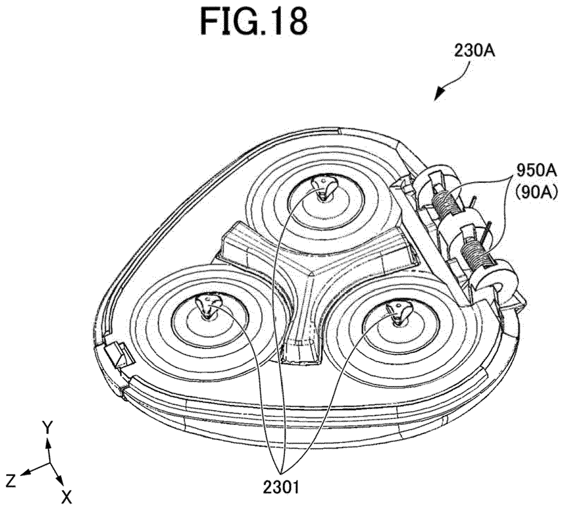

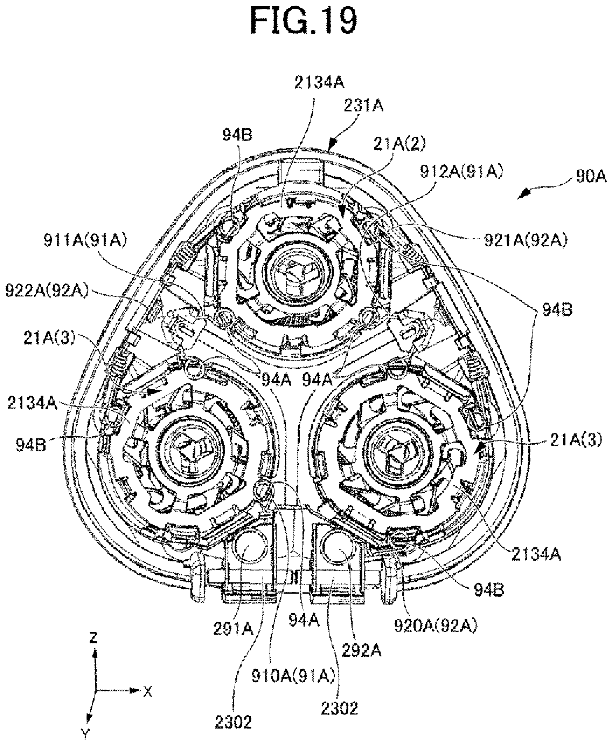

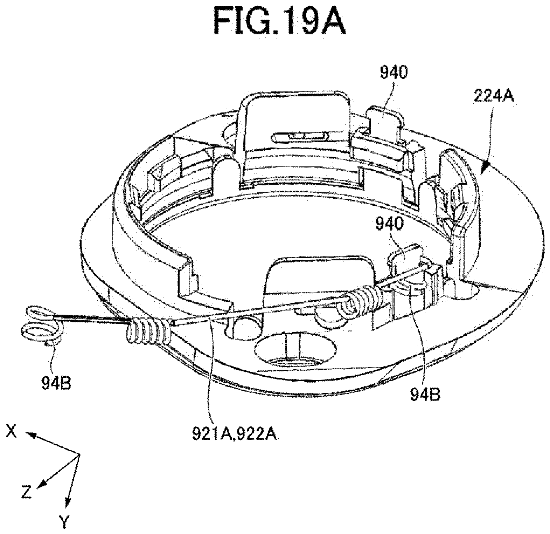

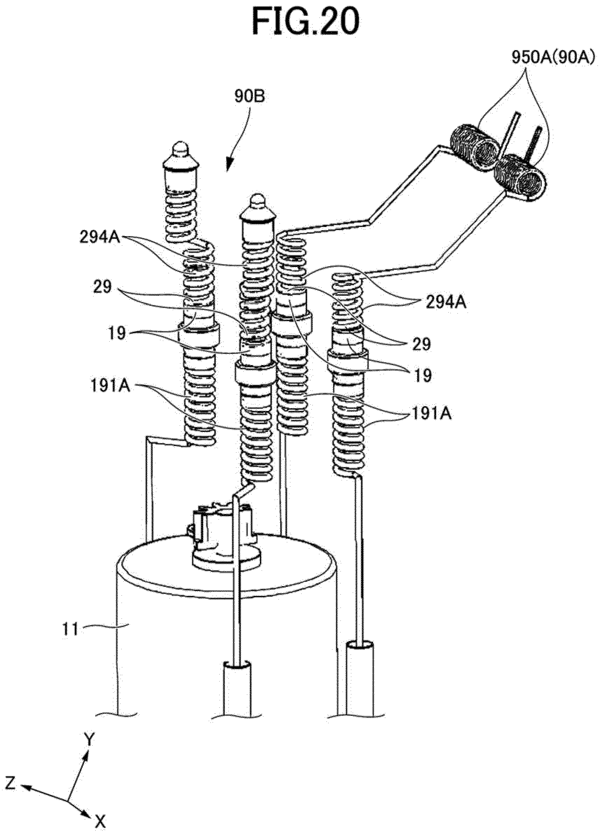

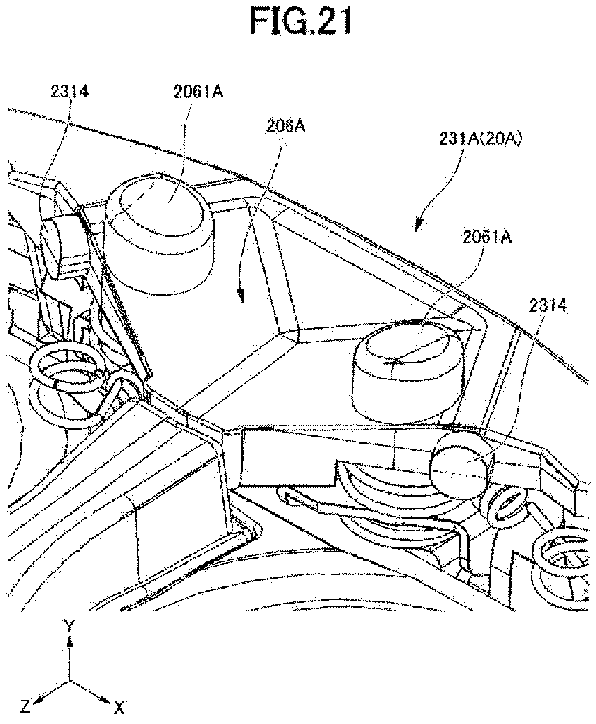

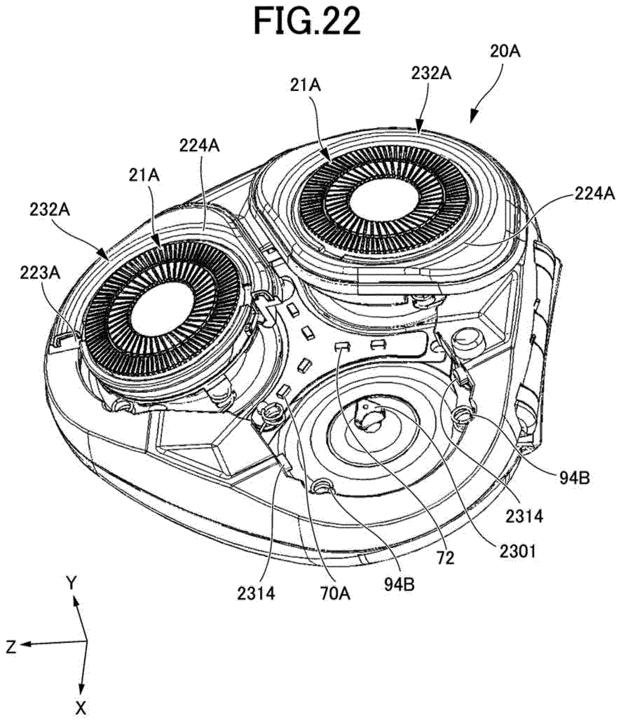

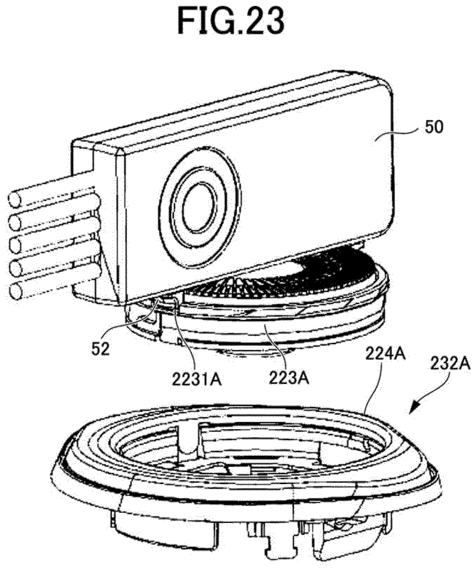

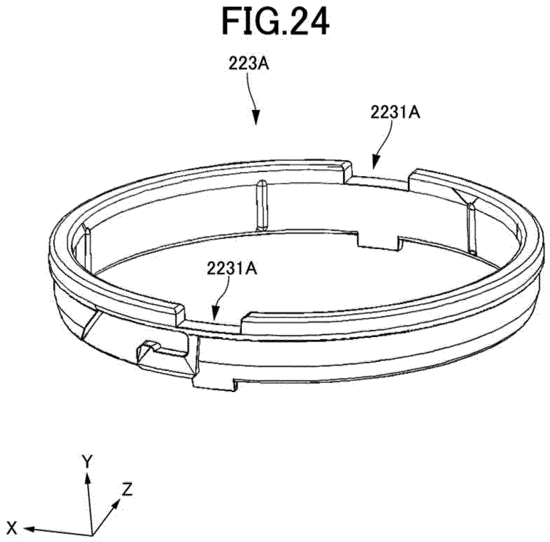

FIG. 16 is a perspective view of the head part 20A of the electric shaver 1A of the present embodiment with cutter units 21A and shaver holders 232A disassembled. FIG. 17 is a perspective view of the head part 20A of the electric shaver 1A of the present embodiment with one cutter unit 21A and one shaver holder 232A removed and one cutter unit 21A and one shaver holder 232A illustrated in a cross-sectional view. FIG. 18 is a perspective view of a base portion 230A of the electric shaver 1A of the present embodiment. FIG. 19 is an explanatory diagram of a wiring structure 90A of the present embodiment, and is a perspective view from below of the head part 20A of the electric shaver 1A of the present embodiment. In FIG. 19, the base portion 230 is removed. FIG. 19A is a perspective view from the lower side of a frame 224A together with part of the wiring structure 90A. FIG. 20 is a perspective view of an electrical connection structure of the electric shaver 1A of the present embodiment. FIG. 21 is an enlarged perspective view of characteristic part of a shaver holder 232A. FIG. 22 is a perspective view illustrating an arrangement of a light irradiation unit 70A, etc. of the electric shaver 1A of the present embodiment. FIG. 23 is an explanatory diagram of a method for detaching the cutter unit 21A of the electric shaver 1A of the present embodiment. FIG. 24 is a perspective view of a cutter unit retaining ring 223A of the electric shaver 1A of the present embodiment.

Also in the present embodiment as in the above-described embodiment, the main holder portion 231A of the head part 20A swingably holds the cutter units 21A and the frames 224A via the shaver holders 232A. Specifically, also in the present embodiment, each shaver holder 232A rotatably supports the cutter unit 21A in such a manner that the cutter unit 21A can swing with respect to the head part 20A, similarly to the shaver holder 232 of the above-described embodiment. That is, also in the present embodiment, the main holder portion 231A has the rotation shaft support portions 2314 extending in a direction perpendicular to the corresponding rotation shaft portion 2301 of the base portion 230A (see FIG. 17) (an exemplary second swing support mechanism), and the main holder portion 231A supports the shaver holders 232A rotatably about the rotation shaft support portions 2314.

In the present embodiment, each frame 224A is provided on an upper-side surface of an outer peripheral portion of the shaver holder 232A, which is unlike the frame 224 according to the above-described embodiment. For example, the frame 224A may be fixed to the shaver holder 232A by fitting or the like, or may be integrally molded by insert molding or the like.

Also in the present embodiment as in the above-described embodiment, the outer blade portion 2131 (the contact surface portion 21311) and the frame 224A of the cutter unit 21A form the pair of electrodes 221 related to the heater 22 (an exemplary operation unit).

In the present embodiment, the head part 20A accommodates the wiring structure 90A that energizes the pair of electrodes 221. The wiring structure 90A is an electrical connection structure that establishes an electrical connection between the pair of electrical contacts 29 and the pairs of electrodes 221 illustrated in FIG. 2.

Specifically, the wiring structure 90A includes a first wiring 91A having one end joined to one of the pair of electrical contacts 29 and a second wiring 92A having one end joined to the other of the pair of electrical contacts 29. The other end of the first wiring 91A is joined to one of the pair of electrodes 221, and the other end of the second wiring 92A is joined to the other of the pair of electrodes 221.

The first wiring 91A includes a first lead-out portion 910A and two first bridge portions 911A, 912A, as illustrated in FIG. 19.

The first lead-out portion 910A is disposed inside of the main holder portion 231A (on a side toward the base portion 230A). As illustrated in FIG. 19, the first lead-out portion 910A is joined to a first electrode 291A whose one end is electrically connected to one of the pair of electrical contacts 29. As illustrated in FIG. 19, the first lead-out portion 910A has the other end electrically connected to the outer blade portion 2131 (see FIG. 17) of the cutter unit 21A (1). In this situation, the first lead-out portion 910A may be electrically connected to a pressure plate 2134A disposed below the outer blade portion 2131 of the cutter unit 21A (1) via an energizing spring 94A (described later).

As illustrated in FIG. 19, the first bridge portion 911A has one end electrically connected to the outer blade portion 2131 of the cutter unit 21A (1) via an energizing spring 94A (described later) and the other end electrically connected to the outer blade portion 2131 of the cutter unit 21A (2) via an energizing spring 94A (described later). In this way, the outer blade portion 2131 of the cutter unit 21A (1) and the outer blade portion 2131 of the cutter unit 21A (2) are electrically connected via the first bridge portion 911A.

As illustrated in FIG. 19, the first bridge portion 912A has one end electrically connected to the outer blade portion 2131 of the cutter unit 21A (2) via an energizing spring 94A (described later) and the other end electrically connected to the outer blade portion 2131 of the cutter unit 21A (3) via an energizing spring 94A (described later). In this way, the outer blade portion 2131 of the cutter unit 21A (2) and the outer blade portion 2131 of the cutter unit 21A (3) are electrically connected via the first bridge portion 921A.

Thus, the first wiring 91A electrically connects one of the pair of electrical contacts 29 to the respective outer blade portions 2131 of the three cutter units 21A each forming one of the pair of electrodes 221 via the first lead-out portion 910A and the two first bridge portions 911A, 912A. This allows the three cutter units 21A forming one of the pair of electrodes 221 to be energized all at once via one of the pair of electrical contacts 29.

Similarly, the second wiring 92A includes a second lead-out portion 920A and two second bridge portions 921A, 922A.

The second lead-out portion 920Ais disposed inside of the main holder portion 231A (on a side toward the base portion 230A). As illustrated in FIG. 19, the second lead-out portion 920A is joined to a second electrode 292A whose one end is electrically connected to the other of the pair of electrical contacts 29. As illustrated in FIG. 19, the second lead-out portion 920A has the other end electrically connected to the frame 224A around the cutter unit 21A (3). In this situation, the second lead-out portion 920A may be electrically connected to electrode portions 940 disposed below the frame 224A around the cutter unit 21A (3) via energizing springs 94B (described later), as illustrated in FIG. 19A.

As illustrated in FIG. 19, the second bridge portion 921Ahas one end electrically connected to the frame 224A around the cutter unit 21A (3) via an energizing spring 94B (described later) and the other end electrically connected to the frame 224A around the cutter unit 21A (2) via an energizing spring 94B (described later). In this way, the frame 224A around the cutter unit 21A(3) and the frame 224A around the cutter unit 21A(2) are electrically connected via the second bridge portion 921A.

As illustrated in FIG. 19, the second bridge portion 922Ahas one end electrically connected to the frame 224A around the cutter unit 21A(2) via an energizing spring 94B (described later) and the other end electrically connected to the frame 224A around the cutter unit 21A(1) via an energizing spring 94B (described later). In this way, the frame 224A around the cutter unit 21A(2) and the frame 224A around the cutter unit 21A(1) are electrically connected via the second bridge portion 922A.

Thus, the second wiring 92A electrically connects the other of the pair of electrical contacts 29 to the respective three frames 224A each forming the other of the pair of electrodes 221 via the second lead-out portion 920A and the two second bridge portions 921A, 922A. This allows the three frames 224A each forming the other of the pair of electrodes 221 to be energized all at once via the other of the pair of electrical contacts 29.

Also in the present embodiment as in the above-described Embodiment 1, the first wiring 91A and/or the second wiring 92A may be composed of a wire made of stainless steel. In addition, the first wiring 91A and/or the second wiring 92A may be plated with nickel or zinc. By plating the surface of stainless steel that has surface corrosion resistance and high electrical resistance due to a passive film with nickel having low electrical resistance, the first wiring 91A and/or the second wiring 92A become rust resistant and capable of easily passing electricity therethrough. The ability to pass electricity on the premise of water washing facilitates the designing. The electrical contacts 19 and/or the electrical contacts 29 may also be composed of plated stainless steel as well. The surfaces of the first wiring 91A and/or the second wiring 92A as well as the electrical contacts 19 and/or the electrical contacts 29 may be made of the same material such as nickel. Degradation due to dissimilar metal contact corrosion can be prevented.

In the present embodiment, the first wiring 91A and the second wiring 92A may be provided with an insulation film on the surfaces thereof or may have a wide separation distance therebetween to prevent a contact and hence an electrical short circuit between the first wiring 91A and second wiring 92A. The entire head part 20A may be downsized while a wall is provided between the wirings to increase the creepage distance.

Further, in the present embodiment, it may be arranged in such a way that the insulation resistance of the first wiring 91A and/or the second wiring 92A is larger than the insulation resistance between the frames 224A and the outer blade portions 2131. For example, the distance may be large. This allows more current to flow toward the frames 224A and the outer blade portions 2131 even when an area between the first wiring 91A and the second wiring 92A and areas between the frames 224A and the respective outer blade portions 2131 are filled with a conductive material such as a liquid including water or a gel. For example, the battery 12 can be downsized while ensuring skin heating with high frequency by arranging such that the insulating resistances of the first wiring 91A and the second wiring 92A are 1 megohm or more when they are dry and 100 ohm or more when they are filled with a gel.

As described above, the wiring structure 90A of the present embodiment includes the energizing springs 94A, 94B (an exemplary expandable and contractible conductor element) at the ends of the first bridge portions 911A, the second bridge portions 921A and the like. The energizing springs 94A, 94B are in the form of a coil spring and may be expandable and contractible in a direction along the rotation shaft portions 2301. The energizing springs 94A each has one end electrically connected to the outer blade portion 2131 of the cutter unit 21A and the other end electrically connected to the electrical contact 29. The energizing springs 94B each has one end electrically connected to the frame 224A and the other end electrically connected to the electrical contact 29 via a wire spring 954A or the like to be described later.

The energizing springs 94A, 94B each need not be in direct contact with the lower surface of the outer blade portion 2131 or the lower surface of the frame 224A of the cutter unit 21A, and may be electrically connected via a member (a conductor member). For example, the energizing springs 94A may each be electrically connected to the outer blade portion 2131 via the pressure plate 2134A, which is a conductor, as illustrated in FIG. 19. The energizing springs 94B may each be electrically connected to the frame 224A via the electrode portion 940, as described above with reference to FIG. 19A.

The energizing springs 94A, 94B are configured and disposed in such a manner that a state in which the energizing springs 94A, 94B are electrically connected to the outer blade portions 2131 of the cutter units 21A and the frames 224Ais maintained even in a state in which any rotation of the cutter units 21A with respect to the main holder portion 231A occurs.

In this case, even when any shaver holder 232A rotates with respect to the main holder portion 231A, the energizing springs 94A, 94B expand and contract correspondingly, so that the electrically connected state between the energizing springs 94A, 94B and the frames 224A and the outer blade portions 2131, which are the pairs of electrodes 221, can be maintained. Thus, in the present embodiment, even in any rotational state of the shaver holders 232A (and, accordingly, the frames 224A and the outer blade portions 2131) with respect to the main holder portion 231, the conductive state between the electrical contacts 29 on the head part 20A and the pairs of electrodes 221 (the frames 224A and the outer blade portions 2131) can be maintained.

The wiring structure 90A further includes conductive torsion springs 950A provided around the opening and closing shafts 2302, as illustrated in FIGS. 18 and 19. The torsion springs 950A are provided in a pair. The torsion springs 950A have one ends electrically connected to the first electrode 291A and the second electrode 292A described above, respectively. As illustrated in FIG. 20, the torsion springs 950A have the other ends connected to the electrical contacts 29 (transmission units 294A described later). According to such a wiring structure 90A, as illustrated in FIG. 19, substantially all of the wiring structure 90A is disposed in the head part 20A (i.e., in the main holder portion 231A on the base portion 230A side). Therefore, when the head part 20A is open, as illustrated in FIG. 18, the wiring structure 90A is not substantially disposed on the base portion 230A. In addition, a member such as the contact retaining portion 290 of Embodiment 1 as described above with reference to FIG. 14 is not required.

A wiring structure 90B (FIG. 20 illustrates part of the wiring structure 90B) that energizes the light irradiation unit 70A and a temperature measurement unit 72A that are disposed on the base portion 230A as illustrated in FIG. 22 may be disposed inside the base portion 230A.

In the present embodiment, the cutter unit retaining ring 223A is provided to the outer peripheral portion of the cutter unit 21A. The cutter unit retaining ring 223A has a function of detachably retaining the cutter unit 21A with respect to the head part 20A. The cutter unit retaining ring 223A holds the cutter unit 21A from the upper side toward the shaver holder 232A in such a way that the cutter unit 21A is sandwiched between the cutter unit retaining ring 223A and the shaver holder 232A to facilitate the arrangement of the wiring structure 90A in the main holder portion 231A. The cutter unit retaining ring 223A is disposed on the outer peripheral side of the cutter unit 21A (the outer peripheral side of the cylindrical peripheral wall portion 21312 of the outer blade portion 2131) to hold the cutter unit 21A from the upper side such that the cutter unit 21A is retained by the shaver holder 232A. The cutter unit retaining ring 223A may be press-fitted into the outer peripheral portion of the cutter unit 21A or may be integrally molded by insert molding. In the case that the cutter unit retaining ring 223A is integrated with the cutter unit 21A, the cutter unit 21A can be easily attached and detached with respect to the head part 20A.

In conventional electric shavers, the cutter unit 21Ais detached toward the inside of the head part 20A when the cutter unit 21A is removed for cleaning or replacement. On the other hand, according to the present embodiment, the wiring is disposed inside the head part 20A (i.e., in the main holder portion 231A) such that the user does not accidentally touch the energizing springs 94 A and 94 B that energize the cutter units 21A and the heaters 22. Each cutter unit 21A is configured to be detached toward the outside instead of toward the inside of the main holder portion 231A (the side toward the base 230A) in which the energizing springs 94A, 94B are disposed when the cutter unit 21Ais removed from the main holder portion 231A for cleaning or replacement. Therefore, the cutter unit 21A can be removed from the shaver holder 232A (and accordingly from the main holder portion 231A) without keeping the main holder portion 231A in an open state with respect to the base portion 230A. This makes it possible to effectively use narrow space inside the head part 20A for arrangement of the energizing springs 94A, 94B since the user's fingers and the like do not hit the energizing springs 94A, 94B during attachment and detachment of the cutter unit 21A. Consequently, it is possible to achieve the above-described skin treatment utilizing energization while improving the usability by downsizing the entire head part 20A.

In addition, in the present embodiment, the cutter unit retaining ring 223A has a substantially cylindrical form and is configured to rotate coaxially with the cutter unit 21A. The cutter unit retaining ring 223A can also function as a cutter unit attaching and detaching unit when the cutter unit 21A is removed from the main holder portion 231 for cleaning and replacement of the cutter unit 21A. Specifically, the cutter unit retaining ring 223A is rotatable with respect to the head part 20A between a fixed angle at which the cutter unit 21A is fixed to the shaver holder 232A (and accordingly to the head part 20A) and a detached angle at which the cutter unit 21A is detached from the shaver holder 232A (and accordingly from the head part 20A). In addition, a groove 2231A is provided on the upper side of the cutter unit retaining ring 223A (the side closer to the skin). The groove 2231A is configured so as to allow a rotational operation within the angular range between the fixed angle and the detached angle.

In conventional electric shavers, the cutter unit attaching and detaching unit that is used when the cutter unit is removed for cleaning or replacement of the cutter unit is configured on the inner side of the head part (the side toward the base) in proximity to the cutter unit such that the cutter unit can be attached and detached from the inside of the head part. On the other hand, in the present embodiment, the cutter unit retaining ring 223A has a cylindrical form that rotates coaxially with the cutter unit 21A and detachably fixes the cutter unit 21A from the upper side of the head part 20A. Further, the cutter unit retaining ring 223A has the groove 2231A that is provided on the upper side (on the top side) of the head part 20A, not on the inner side of the head part 20A. Rotating the groove 2231A clockwise fixes the cutter unit 21A, and rotating the groove 2231A counterclockwise detaches the cutter unit 21A. The cutter unit retaining ring 223A is disposed in such a manner that the contact surface portion 21311 of the cutter unit 21A, the upper-side surface (an effect applying surface) of the frame 224A, and the cutter unit retaining ring 223A are disposed so as to be farther from the user's skin (the upper side) in this order, so that the groove 2231A of the cutter unit retaining ring 223A does not touch the user's skin while the head part 20A abuts the skin. This prevents the cutter unit 21A from being inadvertently detached while ensuring an beneficial effect on the skin via the head part 20A. Additionally, since the cutter unit retaining ring 223A is cylindrical and thin and the gap between the cutter unit 21A and the frame 224A can be narrow, it is possible to reduce electric resistance when energizing therebetween while maximizing the beneficial effect. Therefore, the beneficial effect on the skin can be optimized while the usability is improved by downsizing the head part 20A.

The upper-side surface (the effect applying surface) of the frame 224A preferably changes in height so as to form a gently curved surface from the inner side (the side in contact with the cutter unit retaining ring 223A) toward the outer side in such a way as to be farther from the user's skin. Slits 219 (see FIG. 17) extend from the contact surface portion 21311 to the lateral surface of the cutter unit 21A. The slits 219 guide hair of the user into the cutter unit 21A. The slits 219 may be in the form of a linear hole into which hair can enter. The level difference between the contact surface portion 21311 and the upper-side surface of the frame 224A and the curved surface make it easier for the user's hair to be captured into the cutter unit 21 even in the case of a complex curved surface of the user's skin and make it possible to bring the contact surface portion 21311 and the upper-side surface of the frame 224A into contact with a larger area of the skin. Therefore, the beneficial effect on the skin can be optimized while the usability is improved by downsizing the head part 20A. More preferably, when the level difference between the contact surface portion 21311 and the upper-side surface of the frame 224A is set to be 0.2 mm or more and 2 mm or less, effective shaving performance can be achieved while improving the texture on the user's skin.

Further, in the present embodiment, a cleaner 50 in the form of a brush for cleaning the head part 20Ais provided. At a part of the cleaner 50, a protrusion 52 to face the groove 2231A is provided such that the cutter unit retaining ring 223A can be rotated from the fixed angle to the detached angle.

In conventional electric shavers, there is a case where a protrusion on which the user can easily hook his/her finger is provided to the frame for detachment from the inside of the head part. On the other hand, in the present embodiment, since the protrusion 52 to face the groove 2231A of the cutter unit 21A is provided to the cleaner 50 for cleaning the cutter unit 21A, the cutter unit 21A can be attached and detached with respect to the head part 20 simply by placing the protrusion 52 of the cleaner 50 in the groove 2231A and rotating it. This eliminates the necessity of the protrusion on which the user's finger is hooked as in conventional attaching and detaching units, so that the head can be designed to be small, and thus the usability is improved.

In the present embodiment, the main holder portion 231A of the head part 20A that rotatably supports the shaver holders 232A (and accordingly the cutter units 21A and the frames 224A) is provided with recesses 206A on the upper-side surface thereof as illustrated in FIGS. 17 and 21. The recesses 206A may have, for example, a V-shaped cross section as viewed from the side. The recesses 206A are each formed in a direction perpendicular to the rotation shaft of the rotation shaft support portions 2314. The recesses 206A are provided between a plurality of adjacent shaver holders 232A. The recesses 206A each allow a pair of adjacent shaver holders 232A to fall therebetween toward the adjacent shaver holder 232A side and toward the main holder portion 231A side (the lower side). Each recess 206Amay be provided with a stopper 2061A that stops excessive rotation of the shaver holder 232A (and accordingly the cutter unit 21A and the frame 224A).

In conventional electric shavers, in the case that the head part is provided with shaver holders that swingably support a plurality of cutter units with respect to the head part, it has been the shaver holders that the shape is changed such that the cutter units can abut a convex surface of the skin such as cheeks and a jaw. That is, it has been common that the shaver holders are designed so as to be thinner toward adjacent cutter units and the surface of the adjacent cutter units is designed so as to be concave. However, since such shaver holders are partially thin, the shaver holders cannot be provided with an electrode for skin treatment (e.g., the electrode 221 such as the frame 224A). That is, even if it can be assumed that the electrode 221 can be disposed in the thick portion of the shaver holder, the electrode 221 cannot be disposed in the thin portion. If the shaver holder is thickened such that the electrode 221 can be disposed in the thin portion, there occurs a problem that the cutter unit cannot fit a convex surface of the skin and such clearance inhibits the shaving. On the other hand, in the present embodiment, the upper-side surface of the main holder portion 231A of the head part 20A is provided with the recesses 206A to allow the cutter units 21A to fall toward the adjacent cutter units side and toward the head part 20A. Hence, it is possible to provide the heater 22 such as the electrode 221 in the shaver holder 232A. As a consequence, it is possible to achieve both effective skin treatment as described above and effective shaving performance while improving the usability by downsizing the head part 20A.

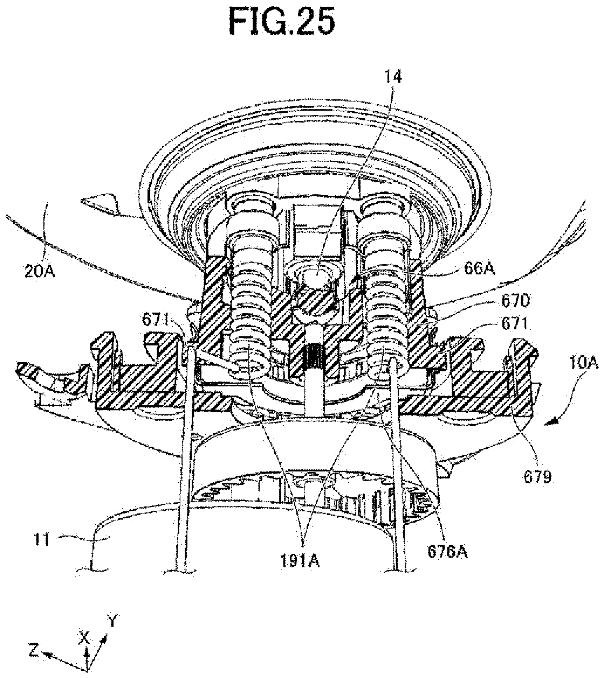

Next, a mechanical connection structure and an electric connection structure between the head part 20A and the body part 10A according to the present embodiment will be described in detail with reference to FIGS. 25 to 27 together with FIG. 20 referred above. The mechanical connection structure and the electric connection structure between the head part 20A and the body part 10A described below can also be applied to various skin treatment devices other than the electric shaver 1A (e.g., facial treatment devices, etc.). Also, the wiring structure 90A described above with respect to FIG. 19, etc. can be applied to various skin treatment devices other than the electric shaver 1A (e.g., facial treatment devices, etc.).

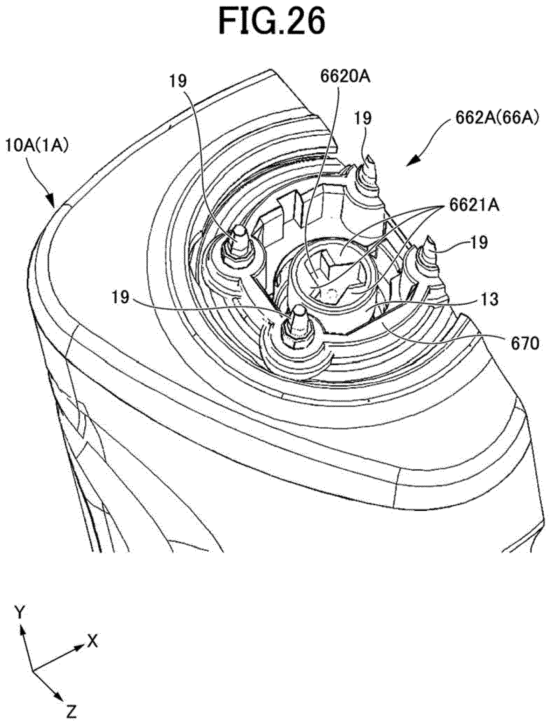

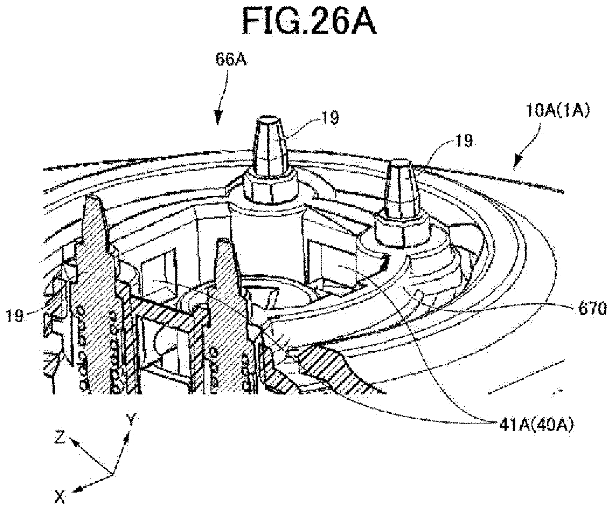

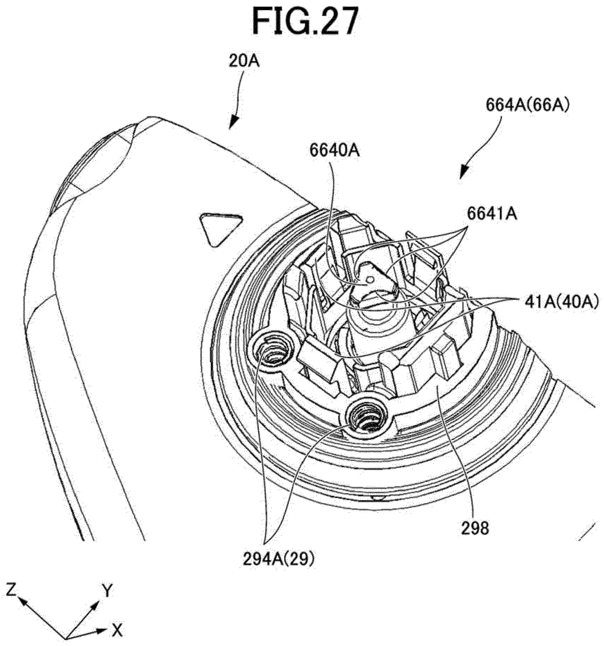

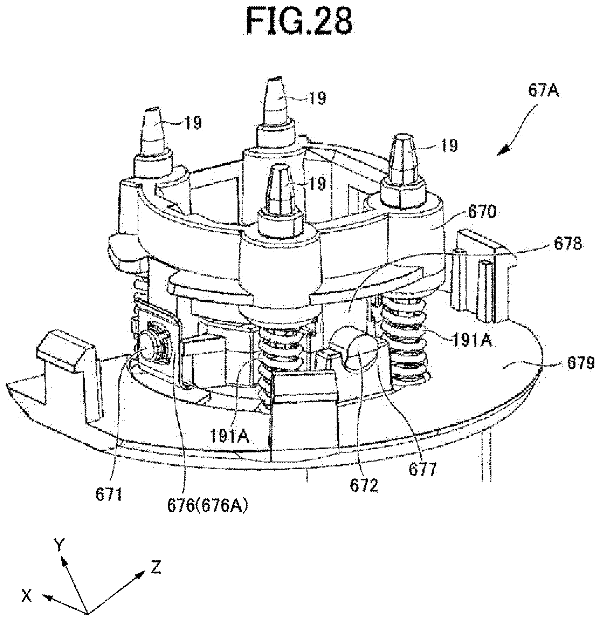

FIG. 25 is a perspective view from below illustrating a connecting part between the head part 20A and the body part 10A of the electric shaver 1A of the present embodiment. FIG. 26 is a perspective view of a connection region of the body part 10A of the electric shaver 1A of the present embodiment, in which part of the body part 10A is cut off. Fig. 26A is a perspective view of the connection region of the body part 10A of the electric shaver 1A of the present embodiment from an angle different from that of FIG. 26. FIG. 27 is a perspective view of a connection region of the head part 20A of the electric shaver 1A of the present embodiment, in which part of the head part 20A is cut off. FIG. 28 is a perspective view of a second coupling unit 67A.

In the present embodiment, the head part 20A is rotatably coupled to the body part 10A by a first coupling unit 66A so as to be swingable with respect to the body part 10A. The first coupling unit 66A may rotatably couple the head part 20A, for example, in such a manner that the head part 20A is rotatable with respect to the body part 10A about two axes perpendicular to the rotation shaft of the electric motor 11.

Specifically, as illustrated in FIGS. 26 and 27, the first coupling unit 66A includes a coupling portion 662A on the body part 10A side and a coupled portion 664A on the head part 20A side. As illustrated in FIG. 26, the coupling portion 662A has a spherical concave surface 6620A formed at an upper end surface of the shaft member 13 from the electric motor 11. Incidentally, a deceleration mechanism (not illustrated) or the like may be provided between the shaft member 13 and the electric motor 11. As illustrated in FIG. 27, the coupled portion 664A has a spherical convex surface 6640A formed on a lower end surface of the rotary shaft 14 for driving the cutter unit 21. In a state in which the head part 20A is attached to the body part 10A, the spherical concave surface 6620A of the coupling portion 662A fits the spherical convex surface 6640A of the coupled portion 664A. In this case, the head part 20A can rotate with respect to the body part 10A via sliding between the spherical surfaces, i.e., the spherical concave surface 6620A of the coupling portion 662A and the spherical convex surface 6640A of the coupled portion 664A.

The coupling between the coupling portion 662A and the coupled portion 664A is provided in such a manner that rotative power from the electric motor 11 is transmitted to the rotary shaft 14 for driving the cutter unit 21 (and accordingly, the rotation shaft portion 2301) (that is, in such a manner that relative rotation about the shaft of the shaft member 13 is restricted). Specifically, as illustrated in FIG. 26, the coupling portion 662A has a non-circular outer shape (an outer shape having three corners 6621A extending radially inward on the basis of the axis of the shaft member 13) as viewed in the axial direction of the shaft member 13 to restrict the range of the spherical concave surface 6620A. As illustrated in FIG. 27, the coupled portion 664A has a non-circular outer shape (an outer shape having three corners 6641A extending radially inward on the basis of the axis of the shaft member 13) as viewed in the axial direction of the shaft member 13 to restrict the range of the spherical convex surface 6640A.

In the present embodiment, the head part 20A is rotatably coupled to the body part 10A by a second coupling unit 67A (an exemplary first swing support mechanism) so as to be swingable with respect to the body part 10A. As illustrated in FIG. 28, the second coupling unit 67A may be formed by two rotation shafts 671, 672 and two bearings 676, 677 which are perpendicular to each other. The rotation shafts 671 and 672 may be perpendicular to the shaft member 13. In this case, as illustrated in FIG. 28, the rotation shaft 671 and the bearing 676 allow the head part 20A to swing with respect to the body part 10A in a direction perpendicular to both the shaft member 13 and the rotation shaft 671. Similarly, the rotation shaft 672 and the bearing 677 allow the head part 20A to swing with respect to the body part 10A in a direction perpendicular to both the shaft member 13 and the rotation shaft 672. According to the swing mechanism related to the second coupling unit 67A, the head part 20A including the surface of the frame 224A (the effect applying surface) is supported with respect to the body part 10A so as to be swingable back and forth and left and right, and the substantial distance therebetween does not vary.

Hence, the user does not need to hold the head part 20A and the body part 10A separately, but only need to hold the body part 10A and lightly place the head part 20A at an approximate position on the skin subject to treatment in order to reliably make the surface of the frame 224A (the effect applying surface) fit the complex contours of the skin. When the user wants strong treatment or stimulation, the use only needs to firmly press the body part 10A toward the skin without touching the head part 20A. Conversely, when the user wants weak treatment or wants to reduce stimulation, the user only needs to lightly press the body part 10A toward the skin.

The two rotation shafts 671, 672 and the respective two bearings 676, 677 may have a gap of 1 mm in the present embodiment in a radial direction about the rotation shafts 671, 672 for ease of rotation. The head part 20A is biased in advance in a direction toward the skin by transmission units 191A (coil springs described later). Therefore, the head part 20A can be move up and down in a direction toward the skin, for example, in a range of about 1 mm. When the user presses the electric shaver 1A against the skin with more force than necessary, the transmission units 191A contract in the range of about 1 mm to absorb the force to prevent the skin from being damaged.

In the present embodiment, the electrical contacts 19 (see FIG. 2) on the body part 10A side each include the transmission unit 191A including a coil spring that is expandable and contractible up and down (for example, in a direction parallel to the axial direction of the shaft member 13). As illustrated in FIG. 20, the transmission units 191A each has one end connected to the body part side and the other end forming the electrical contact 19. In this case, the electrical contacts 19 may be in the form of a conductor piece supported by a coil spring (an exemplary expandable and contractible conductor element) forming the transmission unit 191A. In this case, the conductor piece may be in a cylindrical or columnar form, and may be fixedly supported by the coil spring in such a manner that a part of the conductor piece protrudes from the upper portion of the coil spring forming the transmission unit 191A.

In the present embodiment, the electrical contacts 19 are supported so as to be movable up and down with respect to a contact holder 670, as illustrated in FIGS. 26, 26A, and 28. Specifically, the electrical contacts 19 are each movable up and down with respect to the contact holder 670 with a position at which the upper end surface of the electrode 19 abuts the contact holder 670 as an upper limit position. Each electrical contact 19 is biased upward by the transmission unit 191A including the coil spring described above. Therefore, when the electrical contact 19 is at the upper limit position, the contact holder 670 is biased upward by the transmission unit 191A via the electrical contact 19. The contact holder 670 forms a part of the second coupling unit 67A described above. Specifically, as illustrated in FIG. 28, the contact holder 670 has the rotation shaft 671 and is supported by the bearing 676 of a swing plate 676A attached to a subholder 678. In this case, the subholder 678 has the rotation shaft 672 perpendicular to the rotation shaft 671 and is supported by the bearing 677 on the bracket 679 side. The bracket 679 is fixed to the body part 10A.

In the present embodiment, the second coupling unit 67A includes the two rotation shafts 671 and 672, but the arrangement is not limited thereto. Any other coupling unit may be employed as long as the surface of the frame 224A (the effect applying surface) and the body part 10A can be held at substantially the same distance and the head part 20A and the body part 10A are coupled in a swingable manner. In the present embodiment, the swing mechanism related to the second coupling unit 67A is provided on the body part 10A side, but may be provided on the head part 20A side. In this case, although a contact holder 298 that holds the electrical contacts 29 on the head part 20A side is fixed to the head part 20A, the contact holder 298 may be supported swingably.

In the present embodiment, the electrical contacts 29 (see FIG. 2) on the head part 20A side each include the transmission unit 294A including an expandable and contractible coil spring. Each transmission unit 294A has one end (an end on the upper side) forming the electrical contact 29 of the head part 20A and the other end (an end on the lower side) being a free end. In a state in which the head part 20A is attached to the body part 10A, the end on the lower side of the transmission unit 294A (the lower end of the coil spring) is connected to the upper end of the transmission unit 191A (the electrical contact 19). In this situation, the upper end of the electrical contact 19 in the form of the conductor piece is inserted into the radially inside of the coil spring of the transmission unit 294 such that a conductive state between the electrical contact 19 and the electrical contact 29 can be obtained.

According to such a configuration, even when the head part 20A rotates with respect to the body part 10A, the coils springs of the transmission units 191A and the transmission units 294A expand and contract correspondingly, so that the electric connection between the head part 20A and the body part 10A (that is, the conductive state between the electrical contacts 19 and the electrical contacts 29) can be maintained. That is, in the present embodiment, the electrical connection structure can maintain the electrically connected state between the electrical contacts 19 on the body part 10A side and the electrical contacts 29 on the head part 20A in any state in which the head part 20A swings with respect to the body part 10A.

Thus, according to the present embodiment, owing to the first coupling unit 66A and the second coupling unit 67Athat rotatably couple the head part 20A to the body part 10A (that is, the first coupling unit 66A and the second coupling unit 67A that allow rotation in a manner that does not change the substantial distance between the head part 20A and the body part 10A) as well as the spring contacts (the expandable and contractible transmission units 191A, 294A) disposed in the vicinity of the first coupling unit 66A and the second coupling unit 67A, skin treatment can be carried out with the head part 20A swinging in accordance with concave and convex surfaces the user's skin. That is, since energization to the heater 22, etc. can be continued even if the head part 20A swings, the effective skin treatment as described above can be maintained even if the head part 20A swings in accordance with curved surfaces of the user's skin. Therefore, it is possible to achieve the above-described effective skin treatment while improving the usability of handheld devices.

In the present embodiment, four pairs of the transmission unit 191A and the transmission unit 294A may be provided around the first coupling unit 66A (and a coupling structure 40A described below). In this case, two other pairs of the transmission unit 191A and the transmission unit 294A may be connected to the wiring structure 90B for the light irradiation unit 70A, as illustrated in FIG. 20. This realizes two combinations comprising a combination of positive electrodes and negative electrodes for the heaters 22 and a combination of positive electrodes and negative electrodes for the light irradiation unit 70A. However, in a variant, by providing one pair of the transmission unit 191A and the transmission unit 294A around the first coupling unit 66A, or three or more pairs of the transmission unit 191A and the transmission unit 294A around the first coupling unit 66A, one or three or more sets of a positive electrode and a negative electrode may be disposed.

In the present embodiment, as illustrated in FIG. 22, the light irradiation unit 70A is disposed utilizing a central space surrounded by the three cutter units 21A together with a temperature measurement unit 72A such as a thermistor. In this case, since the head part 20A can be illuminated, the user can perform treatment while constantly checking the state of the skin, so that the user can obtain a desired outcome. Further, the heaters 22 can be appropriately controlled based on measurement results by the temperature measurement unit 72.

In the present embodiment, the head part 20A has the coupling structure 40A that is connected to the head part 20A in such a manner that the head part 20A can be attached and detached with respect to the body part 10A. In this case, it is possible to remove the head part 20A and attach an attachment having another function (an alternative attachment to the head part 20A). In this case, the attachment having another function (e.g., an attachment for a facial treatment device) may comprise a plurality of types of attachments. When the attachment having another function is provided with electrical contacts 29 similar to those of the head part 20A, it can be attached to the body part 10A in a compatible manner. Thus, according to the present embodiment, since the body part 10A can rotatably and detachably hold the head part 20A, it is possible to attach various attachments, which can swing with respect to the skin, and apply various effects on the skin, thereby improving the convenience.

In a configuration that enables attachment (replacement) of various attachments, the control unit 300 of the body part 10A may be capable of identifying (distinguishing) the type of an attachment in an attached state. In this case, the type of the attachment may be identified (distinguished) using a magnetic or electric means such as changing the magnetic resistance value or electrical resistance value related to the electrical connection structure for each type of attachment, or a mechanical means such as changing the shape of the connecting part related to the mechanical connection structure for each type of attachment. Alternatively, the control unit 300 of the body part 10A may identify (distinguish) the type of an attachment in an attached state based on input information from the user. In any case, the control unit 300 can control the output (e.g., the rotational speed of the electric motor 11, photostimulation, thermal stimulation, vibration stimulation, electrical stimulation, etc.) based on the type of the attachment in an attached state to maximize the effect and function thereof.

In the example illustrated, the coupling structure 40A includes a held portion 41A on the head part 20A side and a holding portion 42A on the body part 10A side. In this case, as illustrated in FIG. 27, the held portion 41A may be in the form of a convex claw which may be biased by a plate spring or the like, and the holding portion 42A may be in the form of a concave claw, into which the held portion 41A (in the form of a convex claw) can be fitted, as illustrated in FIG. 26A. In this case, the coupling structure 40A that includes such a fitting structure using claws facilitates attachment and detachment of the head part 20A with respect to the body part 10A.

Further, in the present embodiment, the lower ends of the transmission units 294A on the head part 20A are detachably connected to the upper ends of the transmission units 191A (the electrical contacts 19). That is, when the head part 20A and the body part 10A are in a separated state from each other, the lower-side ends of the transmission units 294A and the upper-side ends of the transmission units 191A are apart from each other and the electrical contacts 19 and the electrical contacts 29 are in a non-conductive state. Therefore, for example, when the head part 20A and the body part 10A are apart from each other, electric wires and the like related to the wiring do not become an obstacle as compared with a configuration in which electric wires and the like maintain a connected state of the electrical contacts 29 and the electrical contacts 19. Thus, the convenience is improved. In addition, attachment (replacement) of various attachments as described above is facilitated, which also improves the convenience.

In the present embodiment, the lower-side ends of the transmission units 294A and the upper-side ends of the transmission units 191A are in a connected state with each other only when the head part 20A is in an attached state in which the head part 20A is attached to the body part 10A. That is, the electrical contacts 19 and the electrical contacts 29 are in a conductive state. Therefore, when the head part 20A and the body part 10A are in a separated state from each other, an operation not intended by a user (malfunction such as an actuation of the heaters 22 of the head part 20A that is in a separated state) can be reliably prevented as compared with a configuration where a state in which the electrical contacts 29 and the electrical contacts 19 are connected is maintained by the wires and the like.

In the present embodiment, the control unit 300 may be capable of distinguishing (detecting) an attached state in which the head part 20A is attached to the body part 10A and a non-attached state. For example, the control unit 300 may distinguish it based on an electrical connection state between the electrical contacts 19 and the electrical contacts 29. In this case, the control unit 300 may be configured to actuate the heaters 22 or the like only while the attached state in which the head part 20A is attached to the body part 10A is being detected.

The exemplary embodiments have been described in detail. However, the present disclosure is not limited to the specific embodiments, and various variations and modifications can be made within the scope of the appended claims. It is also possible to combine all or some of the components of the embodiments described above.

For example, in Embodiment 1 (also in Embodiment 2) described above, the heater 22 is disclosed as an exemplary effect applicator that provides a specific effect to the user's target region 900 that the head part 20 is in contact with. However, the effect applicator that provides a specific effect to the target region 900 of the user is not limited to the heater 22. That is, an effect applicator capable of applying a specific effect other than the heating effect may be used in place of or in addition to the heater 22. In this case, the effect applicator may comprise a pair of electrodes that is similar to the pair of electrodes 221 and generates electrical stimulation to the target region 900 of the user. Also in this case, the effect applicator can operate by receiving power supply from a power source in the manner described above. In this case, the specific effect based on electrical stimulation may include a heating effect by high frequency, an effect related to electrical muscle stimulation (hereinafter EMS), an effect related to iontophoresis, an effect related to ion derivation, or any combination thereof. In this case, EMS advantageously provides tightening and sag care of the target region 900. Ion derivation is advantageously capable of unclogging pores of the skin in the target region 900, and iontophoresis is advantageously capable of moisturizing the skin in the target region 900. These specific effects are suitable as care for skin after shaving.

Further, in Embodiment 2 described above (also in Embodiment 1), electric energy is supplied from the body part 10A to the head part 20A via the transmission units 191A and the transmission units 294A, thereby enabling effective skin treatment such as heating, vibration, light, and electrical stimulation by electricity, but the energy to be supplied is not limited to electric energy. Alternatively to electrical energy, thermal energy or the like may be supplied. In addition, the electrical connection structure implemented via the above-described electrical contacts 19 and electrical contacts 29 may be utilized as a communication line for supplying sensor data (electric signals) from various sensors which may be provided in the head part 20A to the body part 10A. In this case, the various sensors that may be provided in the head part 20A may include a temperature sensor (e.g., the temperature measurement unit 72 described in Embodiment 2 above), a humidity sensor, an image sensor (e.g., an image sensor for measuring a skin condition, etc.), and the like. In addition, the electrical connection structure implemented via the above-described electrical contacts 19 and electrical contacts 29 may be utilized as an electrical power supply line to another element (an element whose power source is electricity other than the light irradiation unit 70A) that may be provided in the body part 10A. In this case, the other element may be a Peltier element, a communication module, or the like.

In Embodiment 2 described above (also in Embodiment 1), the electric shaver 1A is an example of the skin treatment device, but the skin treatment device may be other skin treatment devices such as a facial treatment device as described above. For example, in the case of a facial treatment device, its head part corresponding to the head part 20A may be provided with a plurality of electrodes for applying various types of output waveforms to the skin in place of or in addition to the cutter units 21A. In this case, the electrical connection structure according to Embodiment 2 described above may be used in relation with an electrical connection structure between the plurality of electrodes and the body part 10A. In this case, some or all of the plurality of electrodes may be removably secured to a holder similar to the shaver holder 232A by a retaining ring similar to the cutter unit retaining ring 223A.

### [List of Reference Signs]

- 1, 1A: Electric shaver
- 10: Body part
- 11: Electric motor
- 12: Battery
- 13: Shaft member
- 15: User interface
- 19: Electrical contact (an exemplary contact element)
- 191A: Transmission unit (an exemplary conductive spring element)
- 20, 20A: Head part
- 21, 21A: Cutter unit
- 22: Heater
- 29: Electrical contact
- 294A: Transmission unit (an exemplary conductive spring element)
- 70, 70A: Light irradiation unit
- 230: Base portion
- 2301: Rotation shaft portion
- 2302: Opening and closing shaft
- 231, 231A: Main holder portion (an exemplary head body portion)
- 2310: Opening
- 2314: Rotation shaft support portion (an exemplary second swing support mechanism)
- 232, 232A: Shaver holder (an exemplary second holder portion)
- 2321: Stopper
- 2324: Peripheral wall
- 2131: Outer blade portion
- 21311: Contact surface portion
- 21312: Peripheral wall portion
- 2132: Inner blade portion
- 21321: Cutter blade
- 221: A pair of electrodes
- 224: Frame
- 300: Processor
- 67A: Second coupling unit (an exemplary first swing support mechanism)
- 90: Wiring structure
- 94A: Energizing spring (an exemplary conductive spring element)
- 94B: Energizing spring (an exemplary conductive spring element)
- 900: Target region
- 901: Hair
- 902: Shaving gel

## Claims

1. A skin treatment device, comprising:
a head part configured so as to be capable of making contact with a target region of a user, wherein
the head part has:
an electrically powered cutter unit; and
an effect applicator configured to apply a specific effect to the target region of the user that the head part is in contact with based on electric power supplied from a power source.

2. The skin treatment device according to claim 1, wherein
the effect applicator includes a heating element configured to generate heat by direct current flowing therethrough or a pair of electrodes configured to generate electrical stimulation, and
the specific effect based on the electrical stimulation includes at least one of a heating effect due to high frequency, an effect associated with electrical muscle stimulation, an effect associated with iontophoresis, and an effect associated with ion derivation.

3. The skin treatment device according to claim 2, wherein
the cutter unit has:
an outer blade portion configured so as to be capable of making contact with the target region of the user; and
a plurality of cutter blades with cutting edges, and
one of the pair of electrodes is formed by the outer blade portion.

4. The skin treatment device according to claim 3, wherein
the cutter unit includes a rotary inner blade portion configured so as to be rotatable about a rotation shaft, and
the other of the pair of electrodes is provided around the cutter unit.

5. The skin treatment device according to any one of claims 1 to 4, further comprising:
a body part configured to accommodate an electric motor configured to operate the cutter unit, wherein
the head part is attached to the body part in such a manner as to be detachable and displaceable with respect to the body part.

6. The skin treatment device according to claim 5, wherein the effect applicator is supplied with the electric power from the power source via a contact between an electrical contact provided to the body part and an electrical contact provided to the head part.

7. The skin treatment device according to claim 6, wherein
the electrical contact on the head part is provided to a held portion configured be detachably held by the body part, and
the electrical contact on the body part is provided to a holding portion configured to detachably hold the held portion.

8. The skin treatment device according to claim 6, wherein
the electrical contact on the head part is provided at a position away from a held portion configured to be detachably held by the body part, and
the electrical contact on the body part is provided at a position away from a holding portion configured to detachably hold the held portion.

9. The skin treatment device according to claim 3 or 4, wherein
the head part further has a holder portion configured to displaceably hold the cutter unit,
the one of the pair of electrodes is joined to one end of a wiring whose the other end is electrically connected with a power source, and
the wiring is configured so as to maintain a state in which the wiring is joined to the one of the pair of electrodes even when the cutter unit is displaced with respect to the holder portion.

10. The skin treatment device according to claim 9, wherein
the cutter unit of the head part includes two or more cutter units, and
the wiring includes a wiring portion configured to electrically connect the two or more cutter units to each other.

11. The skin treatment device according to any one of claims 4 to 10, further comprising:
a light irradiation unit provided near the cutter unit and configured to illuminate the target region of the user that the head part is in contact with.

12. The skin treatment device according to any one of claims 1 to 11, wherein the effect applicator is configured to operate in conjunction with electrical rotation of the cutter unit.

13. The skin treatment device according to any one of claims 1 to 11, wherein the effect applicator is configured so as to be operable independently of electrical rotation of the cutter unit.

14. The skin treatment device according to any one of claims 1 to 13, further comprising:
a control unit configured to control the effect applicator.

15. A skin treatment device, comprising:
a body part;
a head part configured so as to be capable of making contact with a target region of a user;
a mechanical connection structure configured to mechanically connect the body part and the head part in an attachable and detachable manner; and
an electrical connection structure configured to electrically connect the body part and the head part.

16. The skin treatment device according to claim 15, wherein the body part and the head part carry out a supply of energy or a signal via the electrical connection structure.

17. The skin treatment device according to claim 15 or 16, wherein the electrical connection structure is electrically disconnected when the head part is detached from the body part.

18. The skin treatment device according to any one of claims 15 to 17, wherein the electrical connection structure includes an expandable and contractible spring element that is conductive.

19. The skin treatment device according to any one of claims 15 to 18, wherein the mechanical connection structure includes a first swing support mechanism configured to support the head part in such a way that the head part can swing with respective to the body part.

20. The skin treatment device according to any one of claims 15 to 19, wherein the head part includes a head body portion and a holder portion configured to hold an operation unit configured to operate based on electrical power,
the head part further includes a second swing support mechanism configured to support the holder portion in such a way that the holder portion can swing with respective to the head body portion,
the electrical connection structure includes a connection structure configured to electrically connect the body part and the operation unit, and
the electrical connection structure maintains a state in which the body part and the operation unit are electrically connected with each other even when the holder portion swings with respect to the head body portion via the second swing support mechanism.
